# EUROPEAN PATENT APPLICATION

(11) **EP 4 248 993 A1**
(43) Date of publication of application: **27.09.2023**
(21) Application number: 22164013.9
(22) Date of filing: 24.03.2022
(51) Int. Cl.: A61K 39/215, C07K 14/165, A61K 39/00

(54) **NEW MERS-COV VACCINE**

(71) Applicant: Charité - Universitätsmedizin Berlin, 10117 Berlin (DE); Max-Delbrück-Centrum für Molekulare Medizin in der Helmholtz-Gemeinschaft, 13125 Berlin (DE)
(72) Inventor: DE LA ROSA, Kathrin, 10437 Berlin (DE); VÁSQUEZ GARCÍA, Clara, 13357 Berlin (DE); SILVIS, Casper, 16321 Bernau bei Berlin (DE); OLSSON, Simon, 42834 Kållered (SE)
(74) Representative: Kador & Partner Part mbB

(57) **Abstract**

The present invention relates to a mutant receptor-binding domain (MERS-mRBD) of MERS-CoV (middle east respiratory syndrome coronavirus) or a fragment thereof and a mutant spike protein (MERS-mSpike) of MERS-CoV or a fragment thereof.

Furthermore, the present invention relates to a mutant spike protein (MERS-mSpike) of MERS-CoV or a fragment thereof comprising the MERS-mRBD or the fragment thereof.

Furthermore, the present invention relates to a polypeptide or protein comprising the MERS-mRBD or the fragment thereof or MERS-mSpike or the fragment thereof and a nucleic acid comprising a nucleotide sequence encoding for the MERS-mRBD or the fragment thereof or the MERS-mSpike or the fragment thereof.

Furthermore, the present invention relates to a vaccine composition comprising one or more MERS-mRBDs or fragments thereof, one or more MERS -mSpikes, one or more polypeptides or proteins and/or one or more nucleic acids according to the present invention.

Furthermore, the present invention relates to the one or more MERS-mRBDs or fragments thereof, the one or more MERS-mSpikes, the one or more polypeptides or proteins, the one or more nucleic acids and/or the vaccine composition according to the present invention for use in the prevention and/or treatment of diseases caused by MERS-CoV in a subject.

## Description

The present invention relates to a mutant receptor-binding domain (MERS-mRBD) of MERS-CoV (middle east respiratory syndrome coronavirus) or a fragment thereof and/or a mutant spike protein (MERS-mSpike) of MERS-CoV or a fragment thereof.

Furthermore, the present invention relates to a mutant spike protein (MERS-mSpike) of MERS-CoV or a fragment thereof comprising the MERS-mRBD or the fragment thereof.

Furthermore, the present invention relates to a polypeptide or protein comprising the MERS-mRBD or the fragment thereof or MERS-mSpike or the fragment thereof and a nucleic acid comprising a nucleotide sequence encoding for the MERS-mRBD or the fragment thereof or the MERS-mSpike or the fragment thereof.

Furthermore, the present invention relates to a vaccine composition comprising one or more MERS-mRBDs or fragments thereof, one or more MERS -mSpikes, one or more polypeptides or proteins and/or one or more nucleic acids according to the present invention.

Furthermore, the present invention relates to the one or more MERS-mRBDs or fragments thereof, the one or more MERS-mSpikes, the one or more polypeptides or proteins, the one or more nucleic acids and/or the vaccine composition according to the present invention for use in the prevention and/or treatment of diseases caused by MERS-CoV in a subject.

Furthermore, the present invention relates to a method for the prevention and/or treatment of diseases, preferably caused by MERS-CoV, preferably Middle East Respiratory Syndrome in a subject

Furthermore, the present invention relates to a method for inducing an immune response, preferably against MERS-CoV in a subject.

MERS-CoV vaccines have not reached market approval yet, despite extensive efforts to develop distinct delivery platforms such as recombinant proteins, virus-like particles, live-attenuated viruses, DNA, as well as vector based vaccines. In recent human clinical trials, neutralizing antibody responses after ChAdOx1 MERS, GLS-5300, and MVA-MERS-S vaccinations were relatively weak and detected in 44-79%, 50%, and 58% of vaccinated study participants, respectively, (https://pubmed.ncbi.nlm.nih.gov/32325038/).

Importantly, the ability of the serum to neutralize the virus depends on neutralizing antibodies (nABs). The induction of antibodies that interfere with MERS-spike interaction are crucial to develop a neutralizing immune response and confer protective immunity. However, activation of B cells and affinity maturation of potent antibodies strongly depends on the availability of the antigen, i.e. the spike proteins or receptor binding domains (RBD) of the spike protein.

MERS-CoV uses CD26 as a host receptor, also known as dipeptidylpeptidase 4 (DPP4) (Wang et al., 2013). The MERS-CoV host receptor CD26/DPP4 binds the virus spike with high affinity and can be released through protease shedding into circulation. In particular, DPP4 binds to the MERS-CoV RBD with reported Kds of 16.7 nM (https://www.nature.com/articles/nature12328) to 57 nM (https://pubmed.ncbi.nlm.nih.gov/26216974). In healthy individuals, serum concentrations of soluble DPP4 were estimated to reach 7 µg/ml (https://onlinelibrary.wiley.com/doi/full/10.1046/j.1365-3083.2001.00984.x?sid=nlm%3Apubmed). Some studies reported concentrations as high as 15 mg/ml DPP4 in healthy subjects (https://pubmed.ncbi.nlm.nih.aov/12233879/) and 0.5-1.5 mg/ml in patients with diabetes (https://pubmed.ncbi.nlm.nih.gov/22153807/). Concentrations of DPP4 in serum have been determined by ELISA or by measuring the catalytic activity of the protein, which is mainly attributed to the dimeric form of this enzyme (https://pubmed.ncbi.nlm.nih.gov/15448155/, https://pubmed.ncbi.nlm.nih.gov/16752891/).

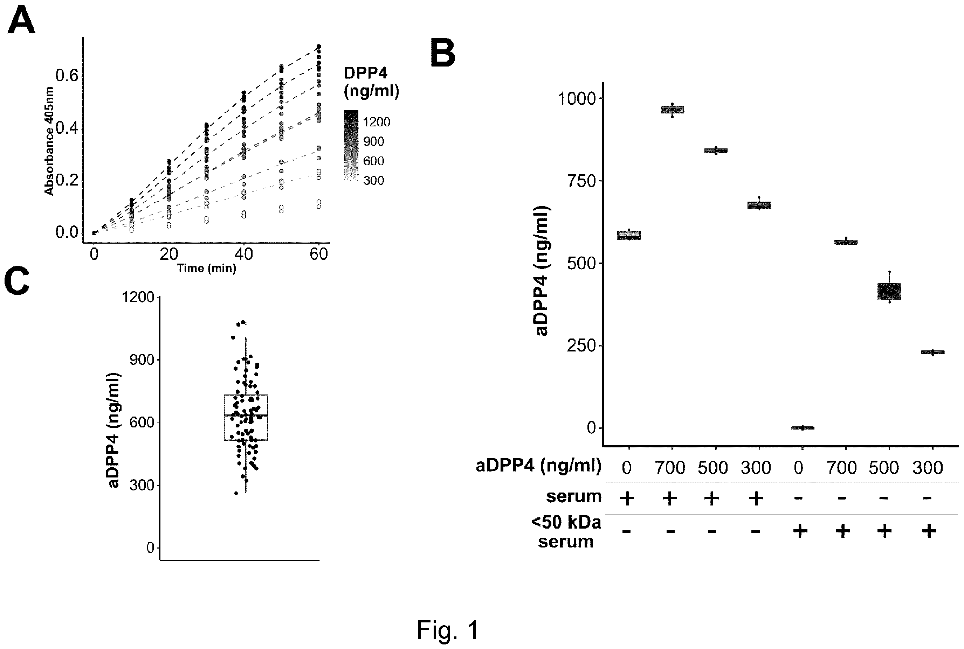

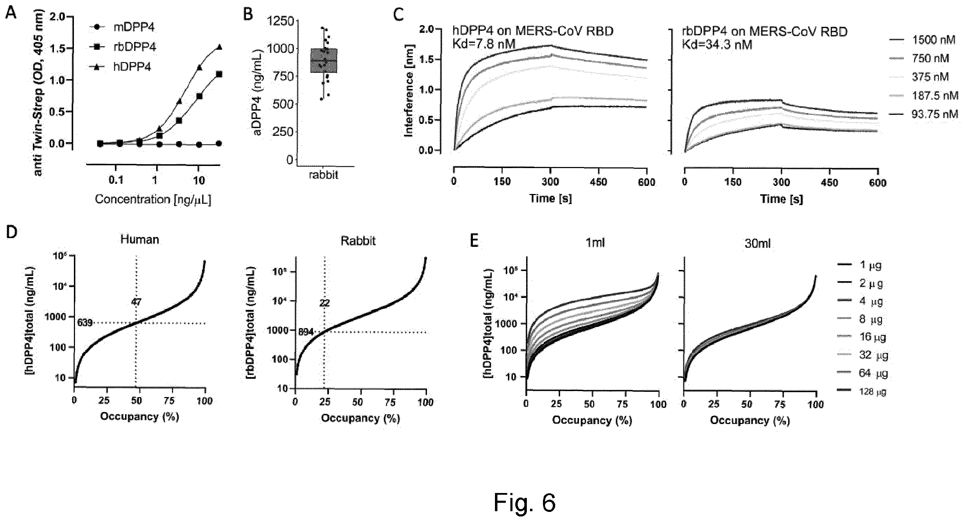

The inventors of the present invention could show that DPP4 levels are high in human blood serum. In particular, the inventors found a median concentration of more than 600 ng/ ml soluble active DPP4 in blood samples (Figure 1). Such concentration bears the risk to occupy the receptor binding domain (RBD), in particular the receptor binding motif (RBM) of the MERS-CoV spike when circulating in the body. Models of antigen occupancy revealed that at a Kd between 7 - 35 nM and soluble DPP4 between 640 - 900 ng/ml, 22 - 47% of the RBD of the MERS-CoV spike would be occupied by DPP4 if 1ug of a MERS-CoV spike protein vaccine would be applied (Figure 6 D-E). The inventors therefore hypothesized that during immune responses, DPP4 could mask the receptor binding domain (RBD) of the spike, thereby interfering with B cell recognition of crucial epitopes. Without being bound to theory, especially subjects with elevated DPP4 may be at risk of developing a less protective immune response. Especially, subjects with increased release of DPP4 could be identified and benefit from the improved vaccine according to the invention. An improved vaccine may not only be relevant before the infection but especially useful when given shortly after infection to promote the development of protective antibodies.

In view of the above, there is an urgent need for novel MERS-CoV vaccines, which overcome the above mentioned problems. It is the object of the present invention to provide novel MERS-CoV vaccines.

The present invention is based on the surprising finding that binding of the receptor DPP4 to the antigen hinders humoral immunity by masking the antigen.

Furthermore, the present invention is based on the surprising finding that all the above mentioned objects can be solved by using a MERS-CoV receptor-binding domain (RBD) or a MERS spike or a fragment thereof for immunization that has a reduced binding strength to its receptor DPP4. In particular, the present invention is based on the surprising finding that the immune responses to vaccines can be improved by reduction or inhibition of binding of the antigen, such as MERS-CoV receptor-binding domain or MERS-CoV spike or a fragment thereof to the host receptor DPP4.

Furthermore, the present invention is based on the surprising finding that a MERS-Cov receptor-binding domain or a MERS-CoV spike or a fragment thereof having reduced binding strength to its receptor DPP4 fostered generation of RBD-binding and/or spike-binding antibodies.

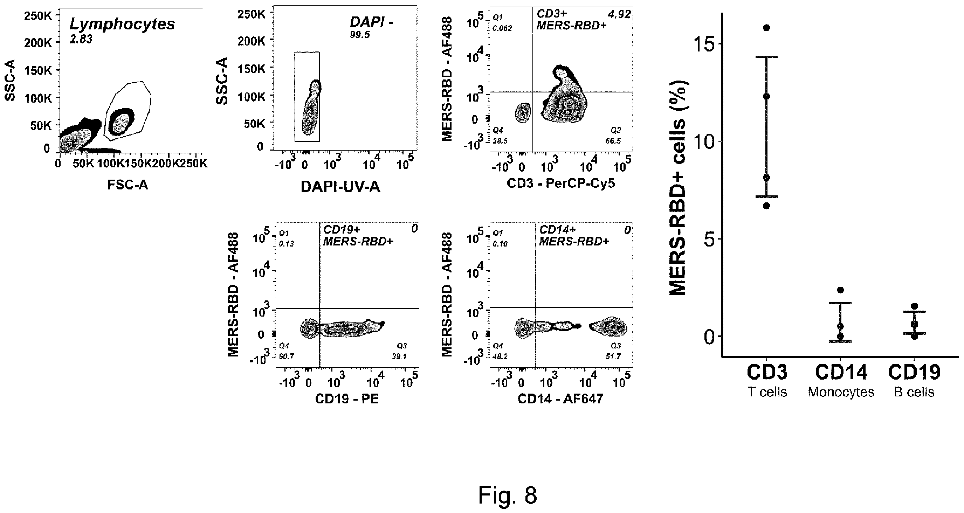

Furthermore, the present invention is based on the surprising finding that a MERS-CoV receptor-binding domain or a MERS-CoV spike or a fragment thereof having reduced binding strength to its receptor DPP4 abrogates masking by DPP4 and fosters generation of RBD-binding and/or spike binding antibodies in presence of the soluble receptor and/or cells expressing the receptor of the surface of the cell (Figure 8).

Furthermore, the present invention is based on the surprising finding that introduction of mutations to a MERS-CoV receptor-binding domain or a MERS-CoV spike or a fragment thereof inhibits or reduces DPP4 binding. It has been further found that introduction of mutations to a MERS-CoV receptor-binding domain or a MERS-CoV spike or a fragment thereof abrogates masking by DPP4. It has been further found that introduction of mutations to a MERS-CoV receptor-binding domain or a MERS-CoV spike or a fragment thereof fosters generation of RBD-binding and/or spike binding antibodies in presence of the soluble receptor and/or cells expressing the receptor of the surface of the cell.

Furthermore, the present invention is based on the surprising finding that the introduction of various single point mutations into the MERS-CoV RBD provides reduced DPP4 binding and fosters generation of RBD-binding and/or spike-binding antibodies.

Furthermore, the present invention is based on the surprising finding that introduction of one or more point mutations in the MERS-CoV RBD of a spike enabling an oligomerization of two or more mSpikes and/or enabling one or more disulphide bonds provides reduced DPP4 binding. In particular, such mutations could lock the RBDs down onto the spike to leave the receptor binding motif inaccessible and could thus be considered as structural mutations. Such combination of mutations in the MERS-CoV RBD of a spike provides reduced DPP4 binding and fosters generation of spike-binding antibodies.

Furthermore, the present invention is based on the surprising finding that introduction of mutations, in particular of single point mutations, in the MERS-CoV RBD of the spike in combination with mutations, in particular single point mutations in the MERS-CoV spike outside the RBD region provides reduced DPP4 binding. In particular, such mutations enable an oligomerization of two or more mSpikes and/or enable one or more disulphide bonds one or more disulphide bonds and could lock the RBDs down onto the spike to leave the receptor binding motif inaccessible and could thus be considered as structural mutations. It has been found that such mutations in the MERS-CoV RBD combined with mutations in the MERS-CoV Spike outside the RBD region provides reduced DPP4 binding and fosters generation of spike-binding antibodies.

Furthermore, the present invention is based on the surprising finding that introduction of mutations, in particular of single point mutations in the MERS-CoV Spike outside the RBD region provides reduced binding to sialic acids. Such mutations in the MERS-CoV Spike outside the RBD region provides reduced sialic acid binding and might therefore foster generation of spike-binding antibodies. The spike protein has a binding cleft for sialic acid which is present on cells. Sialic acid binding may therefore support the attachment of the MERS-antigen to DPP4 expressing cells. Without being bound to theory, reduced sialic acid binding may therefore reduce the attachment of the MERS-antigen to DPP4 expressing cells and fosters generation of spike-binding antibodies. The present invention is further based on the surprising finding that all the above mentioned objects can be solved by using a mutated MERS-CoV RBD (MERS-mRBD), MERS-CoV Spike (MERS-mSpike) or fragments thereof having one or more amino acid substitutions.

Furthermore, the present invention is further based on the surprising finding that all the above mentioned objects can be solved by using mutated MERS-CoV Spike having one or more amino acid substitutions inside and/or outside the RBD region.

Furthermore, the present invention is further based on the surprising finding that all the above mentioned objects can be solved by using a combination of mutated MERS-CoV RBDs having each a different amino acid substitution.

Furthermore, the present invention is based on the surprising finding that all the above mentioned objects can be solved by using a combination of mutated MERS-CoV RBD having each a different amino acid substitution and a mutated MERS-CoV Spike having each a different amino acid substitution.

Therefore, the present invention provides a mutant receptor-binding domain (MERS-mRBD) of MERS-CoV (middle east respiratory syndrome coronavirus) and/or a fragment thereof or mutant spike (MERS-mSpike) of MERS-CoV or a fragment thereof having a reduced binding strength to the RBD-receptor DPP4 (dipeptidylpeptidase 4) of MERS-CoV compared to the wild type receptor-binding domain of MERS-CoV (MERS-wtRBD) and/or having a reduced binding strength to sialic acid compared to a wild type spike of MERS-CoV (MERS-wtSpike).

Hence, the present invention relates to a MERS-mRBD or a fragment thereof having a reduced binding strength to the RBD-receptor DPP4 of MERS-CoV compared to MERS-wtRBD. Preferred embodiments are described in the dependent claims and further in the following and in particular in (A).

Furthermore, the present invention provides a MERS-mSpike or a fragment thereof having a reduced binding strength to the RBD-receptor DPP4 of MERS-CoV compared to MERS-wtRBD. Preferred embodiments are described in the dependent claims and further in the following and in particular in (B).

Furthermore, the present invention provides a MERS-mSpike or a fragment thereof having a reduced binding strength to sialic acid compared to MERS-wtSpike. Preferred embodiments are described in the dependent claims and further in the following and in particular in (B).

Furthermore, the present invention provides a MERS-mSpike or a fragment thereof comprising the MERS-mRBD or the fragment thereof. In such aspect, the mSpike has except for the mutations in the mRBD no further mutations influencing the binding strength to the RBD-receptor DPP4.

Furthermore, the present invention relates to an MERS-mSpike or a fragment according to the invention which further comprises the MERS-mRBD. In particular, in such aspect mutations as described for the mRBD are combined with one or more mutations as described for the mSpike. In such aspect, the mSpike has in addition to the mutations in the mRBD also one or more further mutations which influence the binding strength to the RBD-receptor DPP4 and/or the binding strength to sialic acid.

Furthermore, the present invention provides a polypeptide or protein comprising the MERS-mRBD or the fragment thereof or the MERS-mSpike or the fragment thereof.

Furthermore, the present invention provides a nucleic acid comprising a nucleotide sequence encoding for the MERS-mRBD or the fragment thereof, the MERS-mSpike or the fragment thereof or the polypeptide.

Furthermore, the present invention provides a vaccine composition comprising as an active ingredient one or more MERS-mRBDs or the fragments thereof and/or one or more MERS-mSpikes or the fragments thereof; and/or one or more polypeptides or proteins; and/or one or more nucleic acids.

Furthermore, the present invention provides one or more MERS-mRBDs or the fragments thereof; and/or one or more MERS-mSpikes or the fragments; and/or one or more polypeptides or proteins; and/or one or more nucleic acids; and/or the vaccine composition for use in the prevention and/or treatment of diseases caused by MERS-CoV in a subject.

In particular, the inventors have found that MERS-CoV receptor DPP4, through binding to its antigen mediates masking of crucial epitopes thereby impairing the protective antibody response. This mechanism of epitope masking was so far only described for antibodies but not for viral receptors.

Particularly, the inventors of the present invention have shown for the first time and provided mechanistic evidence that DPP4 interferes with protective immune responses.

The inventors could show for multiple MERS-CoV vaccine designs that abrogate DPP4 binding that recognition by neutralizing antibodies was maintained. They showed that abrogation of DPP4 binding of a MERS-CoV RBD and/or spike based vaccine markedly increased in vivo antibody titers post immunization.

As stated above, the present invention provides a mutant receptor-binding domain (MERS-mRBD) of MERS-CoV (middle east respiratory syndrome coronavirus) or a fragment thereof or mutant spike (MERS-mSpike) of MERS-CoV or a fragment thereof having a reduced binding strength to the RBD-receptor DPP4 (dipeptidylpeptidase 4) of MERS-CoV compared to the wild type receptor-binding domain of MERS-CoV (MERS-wtRBD) and/or having a reduced binding strength to sialic acid compared to a wild type spike of MERS-CoV (MERS-wtSpike).

Thereby, the present invention provides a mutant receptor-binding domain (mRBD) of MERS-CoV (MERSmRBD) or a fragment thereof or mutant spike of MERS-CoV (MERS-mSpike) or a fragment thereof which allows to elicit a highly efficient immune response against MERS-CoV in a subject also in the presence of high levels of soluble DPP4 receptor and/or against MERS-CoV mutants with increased receptor binding ability and thus an increased masking effect.

Preferably, the MERS-mRBD is a mutant receptor-binding domain (mRBD) of a MERS-CoV spike protein or a fragment thereof.

Preferably, the MERS-mRBD or MERS-mSpike or the fragments thereof is a polypeptide or protein, preferably a recombinant polypeptide or protein or a chemically synthesised polypeptide or protein.

Preferably, the DPP4 is a soluble and/or membrane-bound form of the DPP4, preferably the soluble form of the DPP4 (sDPP4).

Preferably, the MERS-mRBD or MERS-mSpike or the fragments thereof comprises, more preferably consists of, an amino acid sequence or a fragment thereof comprising one or more substitutions of amino acid residues in the MERS-wtRBD or the MERS-wtSpike.

The binding strength of the MERS-mRBD or MERS-mSpike or the fragments thereof and of the MERS-wtRBD or MERS-wtSpike to DPP4 can be measured by any method known to the person skilled in the art. For instance, the binding strength can be determined by flow cytometry analysis, e.g. if cells expressing the MERS-mRBD, MERS-mSpike, MERS-wtRBD or MERS-wtSpike on the cell surface are stained with DPP4, directly or indirectly labelled with a fluorophore, or *vice versa* if cells expressing DPP4 on the cell surface are stained with a soluble MERS-mRBD, MERS-mSpike, soluble MERS-wtRBD, or MERS-wtSpike directly or indirectly labelled with a fluorophore, and counting positively stained cells, or by determining the Kd (dissociation constant) or the EC50 (effective concentration with 50% of maximal binding) of the MERS-wtRBD or the MERS-mRBD, or the MERS-wtSpike or the MERS-mSpike respectively, to DPP4 by using an ELISA or Biolayer Interferometry. The reduced binding strength is then obtained by comparing the binding strength of the MERS-wtRBD to DPP4 with the binding strength of the MERS-mRBD to DPP4, or by comparing the binding strength of the MERS-wtSpike to DPP4 with the binding strength of the MERS-mSpike to DPP4.

Preferably, the binding strength is measured by determining the EC50 or the Kd, wherein a higher EC50 or Kd indicates a lower binding strength, or by flow cytometry analysis, wherein a reduction in the counts of positively stained cells indicates a lower binding strength. Consequently, the reduced binding strength is preferably determined by comparing the EC50, Kd and/or the counts of positively stained cells resulting from the binding between the MERS-wtRBD and DPP4 to the EC50, Kd and/or the counts of positively stained cells resulting from the binding between the MERS-mRBD and DPP4, respectively. Respectively, the reduced binding strength is preferably determined by comparing the EC50, Kd and/or the counts of positively stained cells resulting from the binding between the MERS-wtSpike and DPP4 to the EC50, Kd and/or the counts of positively stained cells resulting from the binding between the MERS-mSpike and DPP4, respectively.

Preferably, the EC50 of the MERS-wtRBD, MERS-wtSpike, the MERS-mRBD, or MERS-mSpike to DPP4 is determined as described in 4.c. "Affinity ELISA" and example 3. Preferably, the Kd of the MERS-wtRBD, MERS-wtSpike, the MERS-mRBD, or MERS-mSpike to DPP4 is determined as described in 9. "Biolayer Interferometry", example 5.

In case the reduced binding strength is determined by determining the EC50 or Kd, a reduced binding strength of the MERS-mRBD to DPP4 is preferably indicated by an increase in the EC50 and/or Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the MERS-mRBD is used a vaccine, the binding strength to DPP4 should be reduced as much as possible. Hence, preferably, there is no upper limit for the reduction in the binding strength to DPP4. Hence, preferably there is no upper limit for the increase in the EC50 or Kd. Nevertheless, an upper limit for an increase in the EC50 or Kd may be 500.000-fold or less, more preferably 100.000-fold or less, still more preferably of 10.000-fold or less. Preferably, the EC50 of the MERS-wtRBD or the MERS-mRBD to DPP4 is determined as described in 4.c. Affinity ELISA and example 3.

In case the reduced binding strength is determined by flow cytometry analysis, a reduced binding strength of the MERS-mRBD to DPP4 is preferably indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the MERS-mRBD is used as a vaccine, the binding strength to DPP4 should be reduced as much as possible. Hence, preferably, there is no upper limit for the reduction in the binding strength to DPP4. Hence, preferably there is no upper limit for the reduction in positively stained cells and the reduction may be even 100%. Nevertheless, an upper limit for the reduction in the positively stained cells may be 100 % or less, more preferably 99% or less.

In case the reduced binding strength is determined by Biolayer Interferometry, a reduced binding strength of the MERS-mRBD to DPP4 is preferably indicated by an increase in the Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the MERS-mRBD is used a vaccine, the binding strength to DPP4 should be reduced as much as possible. Hence, preferably, there is no upper limit for the reduction in the binding strength to DPP4. Hence, preferably there is no upper limit for the increase in the Kd. Nevertheless, an upper limit for an increase in the Kd may be 500.000-fold or less, more preferably 100.000-fold or less, still more preferably of 10.000-fold or less. Preferably, the Kd of the MERS-wtRBD or the MERS-mRBD to DPP4 is determined as described in 9.. "Biolayer Interferometry" and example 5.

In case the reduced binding strength is determined by determining the EC50 or Kd, a reduced binding strength of the MERS-mSpike to DPP4 is preferably indicated by an increase in the EC50 and/or Kd of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the MERS-mSpike is used a vaccine, the binding strength to DPP4 should be reduced as much as possible. Hence, preferably, there is no upper limit for the reduction in the binding strength to DPP4. Hence, preferably there is no upper limit for the increase in the EC50 or Kd. Nevertheless, an upper limit for an increase in the EC50 or Kd may be 500.000-fold or less, more preferably 100.000-fold or less, still more preferably of 10.000-fold or less. Preferably, the EC50 of the MERS-wtSpike or the MERS-mSpike to DPP4 is determined as described in 4.c. Affinity ELISA and example 3.

In case the reduced binding strength is determined by flow cytometry analysis, a reduced binding strength of the MERS-mSpike to DPP4 is preferably indicated by a reduction in the counts of positively stained cells of 50% or more, more preferably of 80% or more, even more preferably of 90% or more, still more preferably of 95% or more and still even more preferably of 98% or more. In order to obtain the best effect in vaccination in case the MERS-mSpike is used as a vaccine, the binding strength to DPP4 should be reduced as much as possible. Hence, preferably, there is no upper limit for the reduction in the binding strength to DPP4. Hence, preferably there is no upper limit for the reduction in positively stained cells and the reduction may be even 100%. Nevertheless, an upper limit for the reduction in the positively stained cells may be 100 % or less, more preferably 99% or less.

In case the reduced binding strength is determined by Biolayer Interferomety, a reduced binding strength of the MERS-mSpike to DPP4 is preferably indicated by an increase in the Kd of 1.5-fold or more, more preferably of 2-fold or more, even more preferably of 10-fold or more, still more preferably of 100-fold or more, still even more preferably of 500-fold or more and most preferably of 1000-fold or more. Preferably, a reduced binding strength of the MERS-mSpike to DPP4 is indicated by a Kd of 100 nM or more, more preferably 500 nM or more, more preferably 1 µM or even more preferably 1 mM. In order to obtain the best effect in vaccination in case the MERS-mSpike is used a vaccine, the binding strength to DPP4 should be reduced as much as possible. Hence, preferably, there is no upper limit for the reduction in the binding strength to DPP4. Hence, preferably there is no upper limit for the increase in the Kd. Nevertheless, an upper limit for an increase in the Kd may be 500.000-fold or less, more preferably 100.000-fold or less, still more preferably of 10.000-fold or less. Preferably, the Kd of the MERS-wtSpike or the MERS-mSpike to DPP4 is determined as described in 9 "Biolayer Interferometry" and example 5.

The binding strength of the MERS-mSpike or the fragments thereof and of the MERS-wtSpike to sialic acid can be measured by any method known to the person skilled in the art. For instance, the binding strength can be determined by a hemagglutination assay as described in Wentao Li "Identification of sialic acid-binding function for the Middle East respiratory syndrome coronavirusspike glycoprotein", PNAS September 18, 2017 (https://www.pnas.org/doi/epdf/10.1073/pnas.1712592114). The reduced binding strength is then obtained by comparing the binding strength of the MERS-wtSpike to DPP4 with the binding strength of the MERS-mSpike to sialic acid.

Preferably, the binding strength is measured by determining the HI50, wherein a higher HI50 indicates a lower binding strength. Consequently, the reduced binding strength is preferably determined by comparing the HI50 resulting from the binding between the MERS-wtSpike and sialic acid to the HI50 resulting from the binding between the MERS-mSpike and sialic acid, respectively.

Preferably, the HI50 of the MERS-wtSpike or MERS-mSpike to sialic acid is determined as described in Wentao Li "Identification of sialic acid-binding function for the Middle East respiratory syndrome coronavirusspike glycoprotein", PNAS September 18, 2017 (https://www.pnas.org/doi/epdf/10.1073/pnas.1712592114).

In case the reduced binding strength is determined by determining the HI50 a reduced binding strength of the MERS-mSpike to sialic acid is preferably indicated by an increase in the HI50 of 1.5-fold or more, more preferably of 2-fold or more, even more preferably of 10-fold or more, still more preferably of 100-fold or more, still even more preferably of 500-fold or more and most preferably of 1000-fold or more. In order to obtain the best effect in vaccination in case the MERS-mSpike is used as a vaccine, the binding strength to sialic acid should be reduced as much as possible. Hence, preferably, there is no upper limit for the reduction in the binding strength to sialic acid. Hence, preferably there is no upper limit for the increase in the HI50. Nevertheless, an upper limit for an increase in the HI50 may be 500.000-fold or less, more preferably 100.000-fold or less, still more preferably of 10.000-fold or less.

The RBD is a crucial target for neutralizing antibodies (nAbs). Mutations in the RBD preventing receptor binding may consequently destroy nAb epitopes and decrease the induction of nAbs when the mutant antigen is used for immunizations.

Preferably, the MERS-mRBD, the MERS-mSpike or the fragments thereof exhibits a binding to anti-MERS-wtRBD neutralizing antibodies (anti-MERS-wtRBD-nABs). That is, a binding strength to anti-MERS-wtRBD neutralizing antibodies (anti-MERS-wtRBD-nABs) is preferably maintained, i.e. in the range of an only moderate reduction in the binding strength up to an increase of the binding strength when compared with the MERS-wtRBD.

Hence, preferably, the MERS-mRBD or a fragment thereof and/or a MERS-mSpike of MERS-CoV or a fragment thereof have a reduced binding strength to the RBD-receptor DPP4 of MERS-CoV compared to a MERS-wtRBD and/or have a reduced binding strength to sialic acid and at the same time recognition by neutralizing antibodies is maintained.

The anti-MERS-wtRBD-nABs may belong to different antibody classes like Class I, Class II, Class III and/or Class IV, and/or may be specific for different epitopes in the MERS-wtRBD. The anti-MERS-wtRBD-nABs may be monoclonal and/or polyclonal antibodies reactive against the MERS-wtRBD. Monoclonal anti-MERS-wtRBD-nABs may be obtained recombinantly. Polyclonal anti-MERS-wtRBD-nABs may be obtained from blood plasma or blood serum derived from an immune subject like a convalescent subject, which has been passed through a coronavirus infection, and/or an immunized subject, e.g. immunized by vaccination, wherein such immune subjects are preferably immunologically reactive to the MERS-wtRBD. The subject preferably is a vertebrate, more preferably an immune mammal, more preferably an ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human. Still more preferably the immune subject is a mouse, rat, rabbit, hamster, goat, donkey, horse, dog, cat, sheep or human, etc., most preferably a human.

The binding strength of the MERS-mRBD, MERS-mSpike or the fragment thereof and of the MERS-wtRBD, MERS-wSpike to anti-MERS-wtRBD-nABs can be measured by any method known to the person skilled in the art. For instance, the binding strength can be determined by flow cytometry analysis, e.g. if cells expressing the MERS-mRBD, MERS-wtRBD, MERS-mSpike or MERS-wtSpike on the cell surface are stained with an monoclonal anti-MERS-wtRBD-nAB, directly or indirectly labelled with a fluorophore, or *vice versa* if cells expressing a monoclonal anti-MERS-wtRBD-nAB on the cell surface are stained with a soluble MERS-mRBD, soluble MERS-wtRBD, soluble MERS-mSpike or soluble MERS-wtSpike directly or indirectly labelled with a fluorophore, and counting positively stained cells, or by determining the Kd (dissociation constant) or the EC50 (effective concentration with 50% of maximal binding) of the MERS-wtRBD and the MERS-mRBD or, respectively MERS-spike and MERS-mSpike, to the monoclonal anti-MERS-wtRBD-nAB by using an ELISA, or the ED50 (effective dilution at which 50% of serum antibodies is bound) of polyclonal antibodies of blood plasma or blood serum derived from an immune subject like a convalescent subject, which has been passed through a MERS-CoV infection, and/or an immunized subject, e.g. immunized by vaccination, wherein such immune subjects are preferably immunologically reactive to the MERS-wtRBD. The immune subject is defined as above. Preferably, the EC50 the MERS-wtRBD, MERS-mRBD, MERS-wtSpike or MERS-mSpike to the anti-MERS-wtRBD-nABs is determined as described in 4.c. "Affinity ELISA" and example 4.

In case of determining the binding strength of the anti-MERS-wtRBD-nABs by flow cytometry analysis, the EC50 and/or the Kd, the binding strength preferably is the average binding strength of several different monoclonal anti-MERS-wtRBD-nABs, preferably of four or more different arbitrarily selected monoclonal anti-MERS-wtRBD-nABs. Preferably, the four different monoclonal anti-MERS-wtRBD-nABs are 4C2h, D12, LCA60, and MERS4V2 as described in example 4 and the average binding strength is the average EC50 or average Kd of 4C2h, D12, LCA60, and MERS4V2, respectively.

The maintained binding strength, i.e. the binding strength in the range of an only moderate reduction in the binding strength up to an increase in the binding strength, is then obtained by comparing the binding strength or the average binding strength of the MERS-wtRBD, or MERS-wtSpike respectively, to the anti-MERS-wtRBD-nABs with the binding strength or the average binding strength, respectively, of the MERS-mRBD, or MERS-mSpike respectively, to the anti-MERS-wtRBD-nABs.

Preferably, the binding strength is measured by determining the EC50 or the Kd, wherein a higher EC50 or Kd indicates a lower binding strength, or by flow cytometry analysis or determining the ED50, wherein a reduction in the counts of positively stained cells or a lower ED50, respectively, indicate a lower binding strength. Consequently, the maintained, i.e. whether the binding strength is in the range of an only moderate reduction in the binding strength up to an increase in the binding strength, is preferably determined by comparing the EC50, Kd, the counts of positively stained cells and/or the ED50 resulting from the binding between the MERS-wtRBD, or MERS-wtSpike respectively, and the anti-MERS-wtRBD-nABs to the EC50, Kd, the counts of positively stained cells and/or the ED50 resulting from the binding between the MERS-mRBD, or MERS-mSpike respectively, and the anti-MERS-wtRBD-nABs, respectively.

In the case of determining the binding strength by determining the EC50 or Kd, an only moderate reduction in the binding strength of the MERS-mRBD to the anti-MERS-wtRBD-nABs is preferably indicated by an increase in the EC50 and/or Kd of 3-fold or less, even more preferably of 2-fold or less, still more preferably of 1.5-fold or less and still even more preferably 1.2-fold or less. In the case of determining the binding strength by flow cytometry analysis or determining the ED50, an only moderate reduction in the binding strength of the MERS-mRBD to the RBDanti-MERS-wtRBD-nABs is preferably indicated by a reduction in the counts of positively stained cells and/or in the ED50 of 50% or more, more preferably of 80% or more and even more preferably of 90% or more.

In the case of determining the binding strength by determining the EC50 or Kd, an only moderate reduction in the binding strength of the MERS-mSpike to the anti-MERS-wtRBD-nABs is preferably indicated by an increase in the EC50 and/or Kd of 3-fold or less, even more preferably of 2-fold or less, still more preferably of 1.5-fold or less and still even more preferably 1.2-fold or less. In the case of determining the binding strength by flow cytometry analysis or determining the ED50, an only moderate reduction in the binding strength of the MERS-mSpike to the RBDanti-MERS-wtRBD-nABs is preferably indicated by a reduction in the counts of positively stained cells and/or in the ED50 of 50% or more, more preferably of 80% or more and even more preferably of 90% or more.

When determining the binding strength by the EC50, the Kd or flow cytometric analysis, the binding strength is preferably an average binding strength as defined above. Consequently, the only moderate reduction in the binding strength as defined above is preferably an only moderate reduction in the average binding strength as defined above.

In order to obtain the best effect in vaccination in case the MERS-mRBD or MERS-mSpike is used a vaccine, the binding strength to the anti-MERS-wtRBD-nABs should be as high as possible. Hence, preferably, the reduction in the binding strength to the anti-MERS-wtRBD-nABs should be as moderate as possible and preferably the binding strength of the anti-MERS-wtRBD-nABs to the MERS-mRBD or MERS-mSpike is preferably not reduced or even increased, i.e. EC50 or Kd are not increased and/or the counts of positively of stained cells or the ED50 are not reduced.

Consequently, in the case of determining the binding strength by determining the EC50 or Kd, an increase in the binding strength of the MERS-mRBD to the anti-MERS-wtRBD-nABs is preferably indicated by a increase in the EC50 and/or Kd of 1.2-fold or more, more preferably of 1.5-fold or more, most preferably of 3-fold or more. Furthermore, in the case of determining the binding strength by determining the EC50 or Kd, an increase in the binding strength of the MERS-mSpike to the anti-MERS-wtRBD-nABs is preferably indicated by an increase in the EC50 and/or Kd of 1.2-fold or more, more preferably of 1.5-fold or more, most preferably of 3-fold or more.

As stated above, in order to obtain the best effect in vaccination in case the MERS-mRBD or MERS-mSpike is used as a vaccine, the binding strength to the anti-MERS-wtRBD-nABs should be as high as possible. Hence, preferably, there is no upper limit for the increase in the binding strength to the anti-MERS-wtRBD-nABs. Hence, preferably there is no upper limit for the reduction in the EC50 or Kd and the reduction may be even 100%. Preferably, the upper limit for the increase in the binding strength to the anti-MERS-wtRBD-nABs is indicated by a Kd of 50 pM or lower, more preferably 10 pM or lower or even 1 pM.

In case of determining the binding strength by flow cytometry analysis or ED50, an increased binding strength of the MERS-mRBD to the anti-MERS-wtRBD-nABs is preferably indicated by an increase in the counts of positively of stained cells and/or in the ED50, respectively, by 1.1 -fold or more, more preferably by 1.2-fold or more, even more preferably by 1.5-fold or more, still even more preferably by 2-fold or more. In case of determining the binding strength by flow cytometry analysis or ED50, an increased binding strength of the MERS-mSpike to the anti-MERS-wtRBD-nABs is preferably indicated by an increase in the counts of positively of stained cells and/or in the ED50, respectively, by 1.1-fold or more, more preferably by 1.2-fold or more, even more preferably by 1.5-fold or more, still even more preferably by 2-fold or more.

As stated above, in order to obtain the best effect in vaccination in case the MERS-mRBD or MERS-mSpike is used as a vaccine, the binding strength to the anti-MERS-wtRBD-nABs should be as high as possible. Hence, preferably, there is no upper limit for the increase in the binding strength to the anti-MERS-wtRBD-nABs. Hence, preferably there is no upper limit for the increase in positively stained cells and/or the ED50. Nevertheless, an upper limit for an increase in positively stained cells and/or the ED50 may be 10.000-fold or less, more preferably of 1.000-fold or less and still more preferably 100-fold or less.

When determining the binding strength by the EC50, the Kd or flow cytometric analysis, the binding strength is preferably an average binding strength as defined above. Consequently, the increased binding strength as defined above is preferably an increase in the average binding strength as defined above.

Preferably, the present invention relates to a MERS-mRBD or MERS-mSpike or the fragments thereof, wherein the MERS-mRBD or the mSpike or the fragments thereof comprises, more preferably consists of, an amino acid sequence or a fragment thereof comprising one or more substitutions of amino acid residues of the MERS-wtRBD of SEQ ID NO: 1, or of the MERS-wtSpike of SEQ ID NO: 46.

Preferably, the present invention relates to a MERS-mRBD or MERS-mSpike or the fragments thereof, wherein the MERS-mRBD or the mSpike or the fragments thereof comprises, more preferably consists of, an amino acid sequence or a fragment thereof comprising one or more substitutions of amino acid residues of the MERS-wtRBD of SEQ ID NO: 1, wherein the amino acid residues at position L140 of the MERS-wtRBD of SEQ ID NO: 1 (corresponding to L 506 of the MERS-wtSpike of SEQ ID NO: 46) is not substituted to A.

Preferably, the present invention relates to a MERS-mRBD or MERS-mSpike or the fragments thereof, wherein the MERS-mRBD or the mSpike or the fragments thereof comprises, more preferably consists of, an amino acid sequence or a fragment thereof comprising one or more substitutions of amino acid residues of the MERS-wtRBD of SEQ ID NO: 1, wherein the amino acid residues at position L140 of the MERS-wtRBD of SEQ ID NO: 1 (corresponding to L 506 of the MERS-wtSpike of SEQ ID NO: 46) is not substituted to F.

Preferably, the present invention relates to a MERS-mRBD or MERS-mSpike or the fragments thereof, wherein the MERS-mRBD or the mSpike or the fragments thereof comprises, more preferably consists of, an amino acid sequence or a fragment thereof comprising one or more substitutions of amino acid residues of the MERS-wtRBD of SEQ ID NO: 1, wherein the one or more substitutions of amino acid residues is not selected from any of positions D144, E170, D171 or D173, more preferably is not D144A, E170R; D171K; or D173K of the MERS-wtRBD with SEQ ID NO: 1 (corresponding to D510, E536, D537 or D539 of the MERS-wtSpike of SEQ ID NO: 46).

Preferred relevant positions and amino acid substitutions in a MERS-mRBD or MERS-mSpike which particularly beneficially foster generation of RBM-binding antibodies in presence of the soluble receptor and/or cells expressing the receptor of the surface of the cell are disclosed in the following.

### (A) Mutant receptor-binding domain of a MERS-Cov (MERS-mRBD)

As stated above, the present invention relates to a MERS-mRBD of MERS-CoV or a fragment thereof having a reduced binding strength to DDP4 compared to a MERS-wtRBD.

Preferably, the present invention relates to a MERS-mRBD or the fragments thereof, wherein the MERS-mRBD or the fragments thereof comprises, more preferably consists of, an amino acid sequence or a fragment thereof comprising one or more substitutions of amino acid residues of the MERS-wtRBD of SEQ ID NO: 1. Single point mutations in the MERS-CoV RBD have been identified which are capable of abrogating DPP4 binding.

Hence, preferably, the present invention relates to (A) a MERS-mRBD or a fragment comprising, more preferably consisting of, an amino acid sequence or a fragment thereof comprising one or more substitutions of amino acid residues at positions selected from S94, G96, S132, K136, S138, R139, L140, T 146, E 147, V168, G172, Y174, R176, W187, V189, A190, S191, or S193 of the MERS-wtRBD of SEQ ID NO: 1 (corresponding to S460, G462, S498, K502, S504, R505, L506, T512, E513, V534, G538, Y540, R542, W553, V555, A556, S557, or S559 of the MERS-wtSpike of SEQ ID NO: 46) or the fragment thereof, wherein the MERS-mRBD or the fragment thereof has, except for the substitutions, an amino acid sequence identity of 85% or more to SEQ ID NO: 1.

Preferably, the amino acid residues at position L140 is not substituted to A. Preferably, the amino acid residues at position L140 is not substituted to F.

In the present invention, a substitution of an amino acid in the MERS-mRBD or the fragments thereof may preferably be combined with one or more further amino acid substitutions in the MERS-mRBD or the fragments thereof.

Preferably, the one or more substitutions of amino acid residues comprised in the amino acid sequence of the MERS-mRBD comprises one to three substitutions, more preferably one or two substitutions, more preferably one substitution.

Hence, in a preferred embodiment, the one or more substitutions of amino acids in the MERS-mRBD or the fragment comprise, more preferably consisting of, one to three, or two, preferably one or two, more preferably one substitutions of amino acids.

Preferably, the MERS-mRBD or the fragment thereof has, except for the substitutions, an amino acid sequence identity of 90% or more, more preferably of 95% or more, even more preferably of 98% or more, still even more preferably 99% or more and most preferably of 100% to SEQ ID NO: 1.

The MERS-wtRBD of SEQ ID NO: 1 is a MERS-wtRBD, which for instance is found in the AGN70929.1-variant.

The amino acid sequence identity of the MERS-mRBD to the MERS-wtRBD of SEQ ID NO: 1 as defined above should be low enough to still cover MERS-RBDs of MERS-CoV-variants other than SEQ ID NO 1 in order to allow adaptions of the MERS-mRBD or the fragment thereof thereto or to combinations of the mutations, like substitutions, deletions and insertions, comprised in such variants when compared with SEQ ID NO: 1. Examples for such MERS-CoV-variants and/or the comprised mutations are:
- in the receptor-binding domain (RBD) of the S1 subunit, substitution T424I in 2/8 isolates, and substitutions S459 T in 1/8 isolates;
- in the overlapping RBD/RBM (receptor binding motif) region, substitution W553R, was found in the overlapping RBD/RBM (receptor binding motif) region in 2/8 isolates;
- in the fusion peptide, Q1009 L in hepatad repeat region 1 (HR1), and in the transmembrane (TM) region C1313S (the S2 subunit substitutions included S950 T in the fusion peptide, Q1009 L in hepatad repeat region 1 (HR1), and C1313S in the transmembrane (TM) region); and
- the MERS-CoV-variants listed in the following table (retrieved from https://www.ncbi.nlm.nih.gov/pmc/articles/PMC5165220/):

| GenBank protein ID | Yr isolated | Host | Region | Mutation in MERS-CoV RBD residue: | | | | | | | | | | | | | | | | No. of mutations |
|---|---|---|---|---|---|---|---|---|---|---|---|---|---|---|---|---|---|---|---|---|
| | | | | 400 | 424 | 431 | 434 | 457 | 460 | 482 | 506 | 509 | 510 | 520 | 522 | 529 | 530 | 534 | 582 | |
| AFS88936.1 | 2012 | Human | EMC | K | T | A | A | S | S | A | L | D | D | A | Q | I | V | V | N | 0 |
| AFY13307.1 | 2012 | Human | England | | | | | | | | F | | | | | | | | | 1 |
| AGG22542.1 | 2012 | Human | England | | | | | | | | F | | | | | | | | | 1 |
| AGV083791 | 2012 | Human | KSA | | | | | | | | | G | | | | | | | | 1 |
| AGV08584.1 | 2012 | Human | KSA | | | | | | | | | | | | | | | A | | 1 |
| AHI48528.1 | 2013 | Human | KSA | | | P | | | | V | | | | | | | | | | 2 |
| AHI48733.1 | 2013 | Human | KSA | | | | V | | | | | | | | | | | | | 1 |
| AHC74088.1 | 2013 | Human | Qatar | | | | | | F | | | | | | | | | | | 1 |
| AGV08438.1 | 2013 | Human | KSA | | | | | | | | | | | | | | | | I | 1 |
| AIDSS090.1 | 2014 | Human | KSA | | I | | | | | | | | | | | | | | | 1 |
| AID55095.1 | 2014 | Human | KSA | | I | | | | | | | | | | | | | | | 1 |
| AID55087.1 | 2014 | Human | KSA | | | | | | | | | | | | H | | | | | 1 |
| AKL59401.1 | 2015 | Human | Korea | | | | | | | | | | | | | | L | | | 1 |
| ALB08322.1 | 2015 | Human | Korea | | | | | | | | | | G | | | | | | | 1 |
| ALB08289.1 | 2015 | Human | Korea | | | | | | | | | | | | | T | | | | 1 |
| AHY22545.1 | 2013 | Camel | KSA | N | | | | | | | | | | | | | | | | 1 |
| AHL18090.1 | 2013 | Camel | Egypt | | | | S | | | | | | | | | | | | | 1 |
| AHX00711.1 | 2013 | Camel | KSA | | | | | G | | | | | | | | | | | | 1 |
| AHX00721.1 | 2013 | Camel | KSA | | | | | G | | | | | | | | | | | | 1 |
| AHY22555.1 | 2013 | Camel | KSA | | | | | | | | | | | S | | | | | | 1 |

It shall be mentioned that the mutations relating to the MERS-RBD of the MERS-CoV-variants are indicated in view the position in the MERS-Spike protein. The sequence of the MERS-wtRBD of SEQ ID NO: 1 corresponds to the sequence starting at position 367 of the MERS-wtSpike of SEQ ID NO: 46. Consequently, when 366 amino acids are substracted from a certain amino acid position of the MERS-wtSpike or MERS-mSpike, this results in the corresponding amino acid position of the MERS-wtRBD or MERS-mRBD, respectively. A selection of corresponding amino acid positions in the MERS-wtRBD/MERS-mRBD and the MERS-wtSpike/MERS-mSpike is shown in Table A.

**Table A: Selection of corresponding amino acid positions in the MERS-wtRBD/MERS-mRBD and the MERS-wtSpike/MERS-mSpike**

| **MERS position spike** | **MERS position RBD** |
|---|---|
| S460 | S94 |
| G462 | G96 |
| S498 | S132 |
| K502 | K136 |
| S504 | S138 |
| R505 | R139 |
| L506 | L140 |
| T512 | T146 |
| E513 | E147 |
| V534 | V168 |
| G538 | G172 |
| Y540 | Y174 |
| R542 | R176 |
| W553 | W189 |
| V555 | V189 |
| A556 | A190 |
| S557 | S191 |
| S559 | S193 |

Preferably, for the MERS-mRBD or the fragment thereof, the amino acid residue at the position, K136, T 146, E 147, Y174, or W187, of the MERS-wtRBD of SEQ ID NO: 1 (corresponding to K502, T512, E513, Y540, or W553) is substituted with any of the twenty amino acids A, R, N, D, C, Q, E, G, H; I, L, K, M, F, P, S, T, W, Y, V.

Preferably, for the MERS-mRBD or the fragment thereof, the substitution of the amino acid residue at the position:
- S94 is S94W;
- G96 is selected from G96W, G96Y, G96R, G96F, G96L, G96Q, preferably from G96R, G96L, G96Q ;
- S132 is S132W;
- K136 is selected from K136D, K136E, K136G, K136Y, K136S, K136P;
- S138 is S138W;
- R139 is R139L;
- L140 is selected from L140Y, L140W, L140R, L140H, preferably L140 is L140R;
- T146 is T146A;
- E147 is selected from E147Y, E147W, E147R, E147F;
- V168 is V168W;
- G172 is selected from G172C, G172D, G172E, G172F, G172H, G172I, G172K, G172L, G172N, G172Q, G172R, G172T, G172V, G172W, G172A, G172M, G172P, G172S, G172Y, preferably from G172H, G172W, G172E, G172K, G172L, G172Y, G172Q, G172T, G172R or G172D, more preferably from G172E, G172K, G172L, G172Y, G172Q, G172T, G172R, or G172D, more preferably from G172E or G172K; most preferably from G172K;
- Y174 is Y174C;
- R176 is R176G;
- W187 is selected from W187A, W187C, W187D, W187E, W187G, W187K, W187L, W187M, W187N, W187P, W187Q, W187R, W187S, W187T, W187V;
- V189 is selected from V189W, V189Y, V189F, V189R, V189K, V189A, preferably from V189W, V189Y, V189F, V189R, more preferably V189 is V189Y;
- A190 is A190W;
- S191 is selected from S191Y, S191W, S191F;
- S193 is S193W.

Preferably, the MERS-mRBD or the fragment thereof comprises, more preferably consists of, an amino acid sequence or a fragment thereof comprising one or more substitutions of amino acid residues at positions selected from G96, L140, G172, or V189 of the MERS-wtRBD of SEQ ID NO: 1 (corresponding to G462, L506, G538, or V555 of the MERS-wtSpike of SEQ ID NO: 45), or the fragment thereof.

Preferably, the substitution of the amino acid residue at the position
- G96 is selected from G96R, G96Q, G96L,
- L140 is selected from L140R,
- G172 is selected from G 172H, G172W, G172E, G172K, G172L (20), G172Y (22), G172Q (25), G172T(28), G172R(30) or G172D (33),
- V189 is selected from V189W, V189Y, V189R, V189F

More preferably the MERS-mRBD or the fragment thereof comprises an amino acid sequence or a fragment thereof comprising one or more substitutions of amino acid residues at positions selected from L140, G172, or V189 of the MERS-wtRBD of SEQ ID NO: 1 (corresponding to L506, G538, or V555 of the MERS-wtSpike of SEQ ID NO: 45), or the fragment thereof.

Preferably, the substitution of the amino acid residue at the position
- L140 is selected from L140R,
- G172 is selected from G172E, G172K, G172L (20), G172Y (22), G172Q (25), G172T(28), G172R(30) or G172D (33),
- V189 is selected from V189W, V189Y, V189R, V189F;

More preferably, the substitution of the amino acid residue at the position
- L 140 is L140R;
- G172 is G172E, G172K;
- V189 is V189W, V189Y, V189R, V189F;

More preferably, the MERS-mRBD or the fragment thereof comprises an amino acid sequence or a fragment thereof comprising one or more substitutions of amino acid residues selected from, L140R, G172E, G172K, V189W, V189Y, V189R, V189F,

More preferably, the substitution of the amino acid residue at the position
- L140 is selected from L140R,
- G172 is selected from G172K
- V189 is selected from V189Y.

More preferably, the MERS-mRBD or the fragment thereof comprises an amino acid sequence or a fragment thereof comprising one or more substitutions of amino acid residues selected from L140R, G172K, V189Y. Most preferred is V189Y.

Preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 2 , SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37; SEQ ID NO: 38, SEQ ID NO: 39, SEQ ID NO: 40, SEQ ID NO: 41, SEQ ID NO: 42, SEQ ID NO: 43, SEQ ID NO: 44; or SEQ ID NO: 45, provided that the MERS-mRBD or the fragment thereof has a reduced binding strength to DPP4 as defined above. Preferably, the MERS-mRBD or the fragment thereof further exhibits a binding to anti-MERS-wtRBD neutralizing antibodies as defined above.

More preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 3, SEQ ID NO: 4, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37; SEQ ID NO: 38.

More preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37; SEQ ID NO: 38.

More preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 11, SEQ ID NO: 17, SEQ ID NO: 18, SEQ ID NO: 34, SEQ ID NO: 35, SEQ ID NO: 36, or SEQ ID NO: 37, SEQ ID NO: 38.

More preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 11, SEQ ID NO: 18, SEQ ID NO: 35.

More preferably, the MERS-mRBD or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected of SEQ ID NO: 35. Hence, most preferably, the MERS-mRBD or the fragment thereof comprises, preferably consists of, an amino acid selected of SEQ ID NO: 35.

### (B) Mutant spike protein of MERS-CoV (MERS-mSpike)

As stated above the invention relates to a MERS-mSpike of MERS-CoV or a fragment thereof having a reduced binding strength to the RBD-receptor DPP4 of MERS-CoV compared to a MERS-wtRBD and/or having a reduced binding strength to sialic acid compared to a MERS-wtSpike.

Preferably, the present invention relates to a MERS-mSpike or a fragment thereof, wherein the MERS-mSpike or the fragment thereof comprises, more preferably consists of, an amino acid sequence or a fragment thereof comprising one or more substitutions of amino acid residues of the MERS-wtSpike of SEQ ID NO: 46.

The introduction of mutations at distinct positions, in particular of single point mutations in the MERS-CoV Spike outside the RBD region provides reduced sialic acid binding compared to a MERS-wtRBD. Such mutations in the MERS-CoV Spike outside the RBD region provides reduced binding to cells and fosters generation of spike-binding antibodies. Preferably, the present invention relates to a MERS-mSpike or the fragment thereof comprising, more preferably consists of, an amino acid sequence or a fragment thereof comprising one or more substitutions of amino acid residues at positions outside the RBD of the MERS-wtSpike of SEQ ID NO: 46 wherein the MERS-mSpike or the fragment thereof has, except for the substitutions, an amino acid sequence identity of 80% or more to SEQ ID NO: 46. Preferably, the present invention relates to a MERS-mSpike or a fragment thereof comprising, more preferably consisting of, an amino acid sequence or a fragment thereof comprising one or more substitutions of amino acid residues at positions selected from F39, S133, R307, H91 of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Furthermore, the introduction of one or more substitutions of amino acid residues enabling an oligomerization of two or more mSpike proteins provides reduced binding strength to the RBD-receptor DPP4 compared to a MERS-wtRBD. Preferably, the oligomers are covalently linked. Hence, preferably, such oligomerization may be achieved by one or more covalent bonds, preferably disulphide bonds. Oligomerisation may be determined by any method known to the person skilled in the art, for instance by gel electrophoresis under non-reducing and native conditions.

Hence, preferably, the invention relates to a MERS-mSpike or fragments thereof comprising, preferably consisting of, an amino acid sequence or a fragment thereof, comprising one or more substitutions of amino acid residues of the MERS-wtSpike of SEQ ID NO: 46 or the fragment thereof enabling an oligomerization of two or more mSpikes, preferably covalently linked, and/or enabling one or more covalent bonds, preferably disulphide bonds, wherein the MERS-mSpike or the fragment thereof has, except for the substitutions, an amino acid sequence identity of 80% or more to SEQ ID NO: 46.

Due to the oligomerization and/or the covalent bonds, preferably disulphide bonds, the MERS-mSpike is locked in a so-called RBD-down-conformation.

The lock down of RBDs onto the spike backbone has two functions: i) it stabilizes the protein in a so-called pre-fusion conformation thereby preventing induction of non-functional antibodies raised against the post-fusion conformation, and ii) it prevents or at least decreases the binding to DPP4 as the RBM in this not or at least less accessible.

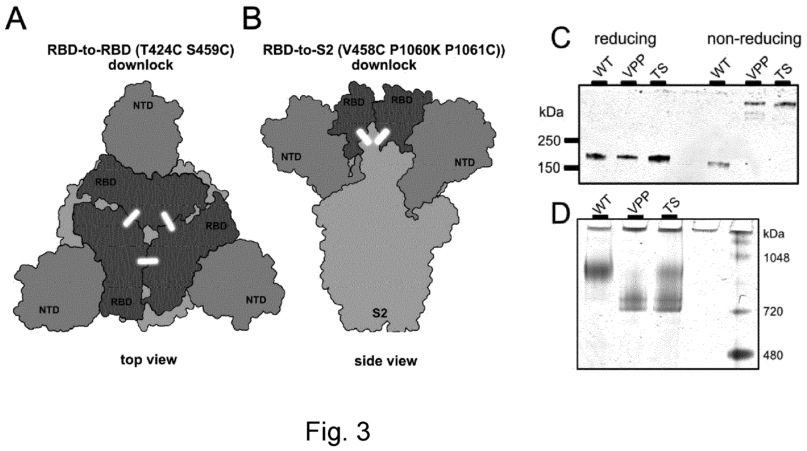

Generally, covalent bonds, preferably disulphide bonds may be formed within a protein, i.e. intramolecular, or between two or more proteins, i.e. intermolecular. Preferably, the one or more substitutions enable disulphide bonds which are intermolecular. Preferably, the one or more substitutions enable the formation of covalent bonds, preferably disulphide bonds between one or more mRBDs and/or between one or more mSpikes. More preferably, the one or more substitutions enable the formation of covalent bonds, preferably disulphide bonds between one or more mRBDs and one or more mSpikes, more preferably, between one or more mRBDs and the S2 unit of one or more mSpikes. Preferably, the oligomerization is a trimerization. Hence, preferably three mSpikes forms a Trimer, preferably the three mSpikes are connected by covalent bonds, preferably disulphide bonds. As an example three mSpikes form a Trimer and the disulphide bonds may be introduced as shown in Figure 3.

Furthermore, such oligomer, preferably trimer, of two or more, preferably three, mSpike proteins provides reduced binding strength of each of the mSpikes or mRBD involved in the oligomer to the MERS-RBD-receptor DPP4. In other words, due to the oligomerization the MERS-mRBD is less or even not accessible to DPP4. Hence, a MERS-mRBD and/or MERS-mSpike involved in the oligomer has at least reduced binding strength to DPP4 compared to a MERS wtRBD.

Preferably, the amino acid sequence comprises at least two substitutions of amino acid residues.

Preferably, a combination of two or more point mutations in the MERS-Spike and/or MERS-RBD enabling an oligomerization of two or more mSpike proteins and/or enabling covalent bonds, preferably disulphide bonds, between two or more MERS-mSpike proteins is used

Hence, preferably, the present invention relates to a MERS-mSpike or the fragment thereof comprising, more preferably consists of, an amino acid sequence or a fragment thereof comprising two or more substitutions of amino acid residues of the MERS-wtSpike of SEQ ID NO: 46 enabling an oligomerization of two or more mSpikes, preferably covalently linked oligomers, and/or enabling one or more covalent bonds, preferably disulphide bonds, preferably between two or more MERS-mSpike proteins, wherein the MERS-mSpike or the fragment thereof has, except for the substitutions, an amino acid sequence identity of 80% or more to SEQ ID NO: 46. In a preferred embodiment, at least two of the two or more substitutions of amino acid residues of the MERS-wtSpike of SEQ ID NO: 46 are at positions inside the RBD. More preferably, the amino acid sequence or a fragment thereof comprises two substitutions which are both at positions inside the RBD of the MERS-wtSpike of SEQ ID NO: 46. In a further preferred embodiment, at least one of the two or more substitutions of amino acid residues of the MERS-wtSpike of SEQ ID NO: 46 is inside the RBD and at least one of the two or more substitutions of amino acid residues of the MERS-wtSpike of SEQ ID NO: 46 is outside the RBD. More preferably, the amino acid sequence or a fragment thereof comprises two substitutions which are both at positions outside the RBD and one substitution which is at a position inside the RBD of the MERS-wtSpike of SEQ ID NO: 46. Preferably, the MERS-mSpike or the fragment thereof is a recombinant polypeptide or protein or a chemically synthesized polypeptide or protein.

Preferably, the MERS-mSpike or the fragment thereof comprises, preferably consists of, an amino acid sequence or a fragment thereof comprising a 2P-mutation and/or an inoperative furin cleavage site, more preferably both, wherein the MERS-mSpike has, except for the 2P mutation and/or the inoperative furin cleavage, an amino acid sequence identity of 80% or more to SEQ ID NO: 46.

The 2P-mutation of the MERS-wtSpike is a substitution of amino acid residues V1060P and L1061 P of the MERS-wtSpike of SEQ ID NO: 46.

The inoperative furin cleavage site is a furin cleavage site, 748-RSVR-751, of the MERS-wtSpike of SEQ ID NO: 46, which is made inoperative by mutation, for instance by substitution, deletion or insertion of amino acid residues, preferably by being substituted with 748-ASVG-751.

Preferably, the MERS-mSpike or the fragment thereof has, except for the 2P-mutation and/or the inoperative furin cleavage site, an amino acid sequence identity of 85% or more, more preferably 90% or more, more preferably of 95% or more, even more preferably of 98% or more, still even more preferably 99% or more and most preferably of 100% to SEQ ID NO: 46.

Preferably, the present invention relates to a MERS-mSpike or a fragment thereof comprising, more preferably consisting of, an amino acid sequence or a fragment thereof comprising one or more substitutions of amino acid residues at positions selected from F39, S133, R307, H91 and/or a combination of three substitutions of amino acid residues at positions V458, V1060 and L1061 (VPP) and/or a combination of two substitutions at positions T424 and S459 (TS) of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Preferably, in case the MERS-mSpike or a fragment thereof comprising, more preferably consisting of, an amino acid sequence or a fragment thereof comprising a combination of three substitutions of amino acid residues at positions V458, V1060 and L1061 (VPP), the amino acid sequence does not comprise the 2P-mutation.

Preferably, the MERS-mSpike or the fragment thereof has, except for the substitutions, an amino acid sequence identity of 85% or more, more preferably 90% or more, more preferably of 95% or more, even more preferably of 98% or more, still even more preferably 99% or more and most preferably of 100% to SEQ ID NO: 46.

The amino acid sequence identity of the MERS-mSpike to the MERS-wtSpike of SEQ ID NO: 46, preferably except for the 2P-mutation and/or the inoperative furin cleavage site; and/or the amino acid sequence identity of the MERS-mSpike to the MERS-wtSpike of SEQ ID NO: 46, except for the substitutions and preferably the 2P-mutation and/or the inoperative furin cleavage site, should be low enough to still cover MERS-Spikes of MERS-CoV-variants other than SEQ ID NO: 46 in order to allow adaptions of the MERS-mSpike thereto or to combinations of the mutations, like substitutions, deletions and insertions, comprised in such variants when compared with SEQ ID NO: 46. Examples for such existing MERS-CoV-variants and/or the comprised mutations are disclosed above under (A).

As explained above the sequence of the MERS-wtRBD of SEQ ID NO: 1 corresponds to the sequence starting at position 367 of the MERS-wtSpike of SEQ ID NO: 46. Consequently, when 366 amino acids are subtracted from a certain amino acid position of the MERS-wtSpike or MERS-mSpike, this results in the corresponding amino acid position of the MERS-wtRBD or MERS-mRBD, respectively. A selection of corresponding amino acid positions in the MERS-wtRBD/MERS-mRBD and the MERS-wtSpike/MERS-mSpike is shown in Table A above.

In the present invention, a substitution of an amino acid in the MERS-mSpike or the fragments thereof may preferably be combined with one or more further amino acid substitutions in the MERS-mSpike or the fragments thereof.

Preferably, the one or more substitutions of amino acids in the MERS-mSpike or the fragment thereof at positions selected from F39, S133, R307, H91 of the MERS-wtSpike of SEQ ID NO: 46 comprise, more preferably consisting of, one to three, or two, preferably one or two, more preferably one substitutions of amino acids.

Preferably, the one or more substitutions of amino acids in the MERS-mSpike or the fragment thereof enabling an oligomerization of two or more mSpikes, and/or enabling one or more covalent bonds, preferably disulphide bonds, comprise, more preferably consisting of, one to three, preferably two or three substitutions of amino acids.

Preferably, the one or more substitutions of amino acids in the MERS-mSpike or the fragment thereof at positions selected from F39, S133, R307, H91 may be combined with one or more substitutions of amino acid residues enabling an oligomerization of two or more mSpikes, and/or enabling one or more covalent bonds, preferably disulphide bonds.

Hence, preferably, the one or more substitutions of amino acids in the MERS-mSpike or the fragment thereof at positions selected from F39, S133, R307, H91 may be combined with a combination of three substitutions of amino acid residues at positions V458, V1060 and L1061 (VPP) of the MERS-wtSpike of SEQ ID NO: 46. Hence, preferably, the MERS-mSpike or the fragment thereof comprises more preferably consists of, an amino acid sequence or a fragment thereof comprising one or more substitutions of amino acid residues at positions selected from F39, S133, R307, H91 and a combination of three substitutions of amino acid residues at positions V458, V1060 and L1061 (VPP) of SEQ ID NO: 46 or the fragment thereof. More preferably, the MERS-mSpike or the fragment thereof comprises more preferably consists of, an amino acid sequence or a fragment thereof comprising one substitution of amino acid residues at any of positions selected from F39, S133, R307, H91 and a combination of three substitutions of amino acid residues at positions V458, V1060 and L1061 (VPP) of SEQ ID NO: 46 or the fragment thereof.

Preferably, the one or more substitutions of amino acids in the MERS-mSpike or the fragment thereof at positions selected from F39, S133, R307, H91 may be combined with a combination of two substitutions at positions T424 and S459 (TS) of the MERS-wtSpike of SEQ ID NO: 46. Hence, preferably, the MERS-mSpike or the fragment thereof comprises more preferably consists of, an amino acid sequence or a fragment thereof comprising one or more substitutions of amino acid residues at positions selected from F39, S133, R307, H91 and a combination of two substitutions at positions T424 and S459 (TS) of the MERS-wtSpike of SEQ ID NO: 46 or the fragment thereof. More preferably, the MERS-mSpike or the fragment thereof comprises more preferably consists of, an amino acid sequence or a fragment thereof comprising one substitution of amino acid residues at any of positions selected from F39, S133, R307, H91 and a combination of two substitutions at positions T424 and S459 (TS) of the MERS-wtSpike of SEQ ID NO: 46 or the fragment thereof.

Preferably also for combinations of substitutions of amino acids the MERS-mSpike or the fragment thereof has, except for the substitutions, an amino acid sequence identity of 80% or more, more preferably 85% or more, more preferably 90% or more, more preferably of 95% or more, even more preferably of 98% or more, still even more preferably 99% or more and most preferably of 100% to SEQ ID NO: 46 as described above.

Preferably, for the MERS-mRBD or the fragment thereof, the amino acid residue at the position, F39, S133, R307, or H91 of the MERS-wtRBD of SEQ ID NO: 46 is substituted with any of the twenty amino acids A, R, N, D, C, Q, E, G, H; I, L, K, M, F, P, S, T, W, Y, V.

Preferably, the substitution of the amino acid residue at the position:
- F39 is F39A
- S133 is S133A
- R307 is R307A
- H91 is H91A
- V458 is V458C
- V1060 is V1060K
- L1061 is L1061C
- T424 is T424C
- S459 is S459C.

Preferably, the MERS-mSpike or the fragment thereof comprises an amino acid sequence or a fragment thereof comprising a combination of substitutions of amino acid residues at positions V458, V1060 and L1061 (VPP) or a combination of substitutions of amino acid residues at positions T424 and S459 (TS) of the MERS-wtSpike of SEQ ID NO: 46 or the fragment thereof.

Preferably, the substitution of the amino acid residue at the position
- V458 is V458C
- V1060 is V1060K
- L1061 is L1061C
- T424 is T424C
- S459 is S459C.

Still more preferably, the MERS-mSpike or the fragment thereof comprises an amino acid sequence or a fragment thereof comprising a combination of substitutions of amino acid residues at positions T424 and S459 (TS) of the MERS-wtSpike of SEQ ID NO: 46 or the fragment thereof.

Preferably, the substitution of the amino acid residue at the position
- T424 is T424C
- S459 is S459C.

Still more preferably, the MERS-mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, provided that the MERS-mSpike or the fragment thereof has a reduced binding strength to DPP4 and/or has a reduced binding strength to sialic acid as defined above. Preferably, the MERS-mSpike or the fragment thereof further exhibits a binding to anti-MERS-wtRBD neutralizing antibodies as defined above.

Notably, all MERS-mSpike sequences provided with the sequence listing (SEQ ID 47 to SEQ ID: 100 and SEQ ID 147 to SEQ ID: 153) include the inoperative furin cleavage site. Furthermore, all MERS-mSpike sequences provided with the sequence listing, except SEQ ID: 47, include the 2P-mutation.

Still more preferably, the MERS-mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 47, or SEQ ID NO: 48.

Still more preferably, the MERS-mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 47.

### Mutant spike protein of MERS-CoV (MERS-mSpike) comprising MERS-mRBD of (A)

As stated above, the present invention preferably relates to a MERS-mSpike or a fragment thereof comprising the MERS-mRBD or the fragment thereof having a reduced binding strength to the RBD-receptor DPP4 of MERS-CoV compared to a MERS-wtRBD according to the present invention. Hence, in this aspect preferably the mSpike does not comprise any of the mutations as described for the mSpike of the invention as in particular described in (B). Such a combination of mRBD of the invention, in particular as described in (A) and mSpike according to the invention, in particular as described in (B) is further described below under *"Mutant spike protein of MERS-CoV (MERS-mSpike) combining mutations described in (A) and (B)".*

In the following the substitutions in the mSpike are disclosed as the amino acid positions of the MERS-wtSpike. Reference is again made to table A showing a selection of corresponding amino acid positions in the MERS-wtRBD/MERS-mRBD and the MERS-wtSpike/MERS-mSpike.

It is to be understood that any embodiment disclosed for the MERS-mRBD or the fragment thereof, in particular under (A) is *mutatis mutandis* applicable to the mSpike comprising the MERS-mRBD or the fragment thereof.

Preferably, the MERS-mSpike or the fragment thereof comprising the MERS-mRBD or the fragment thereof is a recombinant polypeptide or protein or a chemically synthesized polypeptide or protein.

Preferably, the MERS-mSpike or the fragment thereof comprises, preferably consists of, the MERS-mSpike or a fragment thereof, wherein the MERS-spike comprises the MERS-mRBD or the fragment thereof according to the present invention.

Preferably, the MERS-mSpike or the fragment thereof comprises, preferably consists of, an amino acid sequence or a fragment thereof comprising the MERS-mRBD or the fragment thereof according to the present invention and, preferably, a 2P-mutation and/or an inoperative furin cleavage site, more preferably both, wherein the MERS-mSpike has, except for the MERS-mRBD or the fragment thereof and preferably the 2P mutation and/or the inoperative furin cleavage, an amino acid sequence identity of 85% or more to SEQ ID NO: 46.

The 2P-mutation of the MERS-wtSpike is a substitution of amino acid residues V1060P and L1061P of the MERS-wtSpike of SEQ ID NO: 46.

The inoperative furin cleavage site is a furin cleavage site, 748-RSVR-751, of the MERS-wtSpike of SEQ ID NO: 46, which is made inoperative by mutation, for instance by substitution, deletion or insertion of amino acid residues, preferably by being substituted with 748-ASVG-751.

Preferably, the MERS-mSpike or the fragment thereof has, except for the MERS-mRBD or the fragment thereof and preferably the 2P-mutation and/or the inoperative furin cleavage site, an amino acid sequence identity of 85% or more, more preferably of 90% or more, more preferably of 95% or more, even more preferably of 98% or more, still even more preferably 99% or more and most preferably of 100% to SEQ ID NO: 46.

Preferably, the MERS-mSpike or the fragment thereof comprises, preferably consists of, an amino acid sequence or a fragment thereof comprising one or more substitutions of amino acid residues at positions selected from S460, G462, S498, K502, S504, R505, L506, T512, E513, V534, G538, Y540, R542, W553, V555, A556, S557, or S559 of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Preferably, the MERS-mSpike or the fragment thereof has, except for the substitutions, an amino acid sequence identity of 85% or more, more preferably 90% or more, more preferably 95% or more, more preferably of 98% or more, even more preferably of 99% or more and most preferably of 100% to SEQ ID NO: 46.

The amino acid sequence identity of the MERS-mSpike to the MERS-wtSpike of SEQ ID NO: 46, except for the MERS-mRBD or the fragment thereof and preferably the 2P-mutation and/or the inoperative furin cleavage site; and/or the amino acid sequence identity of the MERS-mSpike to the MERS-wtSpike of SEQ ID NO: 46, except for the substitutions and preferably the 2P-mutation and/or the inoperative furin cleavage site, should be low enough to still cover MERS-Spikes of MERS-CoV-variants other than SEQ ID NO: 46 in order to allow adaptions of the MERS-mSpike thereto or to combinations of the mutations, like substitutions, deletions and insertions, comprised in such variants when compared with SEQ ID NO: 46. Examples for such existing MERS-CoV-variants and/or the comprised mutations are disclosed above.

Preferably, for the MERS-mSpike or the fragment thereof, the amino acid residue at the position K502, T512, E513, Y540, or W553 of the MERS-wtSpike of SEQ ID NO: 46 is substituted with any of the twenty amino acids A, R, N, D, C, Q, E, G, H; I, L, K, M, F, P, S, T, W, Y, V.

Preferably, for the MERS-mSpike or the fragment thereof, the substitution of the amino acid residue at the position:
- S460 is S460W;
- G462 is selected from G462W, G462Y, G462R, G462F, G462L, G462Q, preferably from G462R, G462L, G462Q ;
- S498 is S498W;
- K502 is selected from K502D, K502E, K502G, K502Y, K502S, K502P
- S504 is S504W;
- R505 is R505L;
- L506 is selected from L506Y, L506W, L506R, L506H, preferably L506 is L506R;
- T512 is T512A;
- E513 is selected from E513Y, E513W, E513R, E513F;
- V534 is V534W;
- G538 is selected from G538C, G538D, G538E, G538F, G538H, G538I, G538K, G538L, G538N, G538Q, G538R, G538T, G538V, G538W, G538A, G538M, G538P, G538S, G538Y, preferably from G538H, G538W, G538E, G538K, G538L, G538Y, G538Q, G538T, G538R or G538D, more preferably from G538E, G538K; G538L, G538Y, G538Q, G538T, G538R, or G538D, more preferably from G538E or G538K; most preferably from G538K;
- Y540 is Y540C;
- R542 is R542G;
- W553 is selected from W553A, W553C, W553D, W553E, W553G, W553K, W553L, W553M, W553N, W553P, W553Q, W553R, W553S, W553T, W553V;
- V555 is selected from V555W, V555Y, V555F, V555R, V555K, V555A, preferably from V555W, V555Y, V555F, V555R, more preferably V555 is V555Y;
- A556 is A556W;
- S557 is selected from S557Y, S557W, S557F;
- S559 is S559W.

Preferably, the MERS-mRBD or the fragment thereof comprises, more preferably consists of, an amino acid sequence or a fragment thereof comprising one or more substitutions of amino acid residues at positions selected from G96, L140, G172, or V189 of the MERS-wtRBD of SEQ ID NO: 1 (corresponding to G462, L506, G538, or V555 of the MERS-wtSpike of SEQ ID NO: 45), or the fragment thereof.

Preferably, the substitution of the amino acid residue at the position
- G462 is selected from G462R, G462Q, G462L ;
- L506 is selected from L506R;
- G538 is selected from G538H, G538W, G538E, G538K, G538L, G538Y, G538Q, G538T, G538R or G538D;
- V555 is selected from V555W, V555Y, V555R, V555F.

More preferably the MERS-mRBD or the fragment thereof comprises an amino acid sequence or a fragment thereof comprising one or more substitutions of amino acid residues at positions selected from L506, G538, or V555 of the MERS-wtRBD of SEQ ID NO: 1 (corresponding to L506, G538, or V555 of the MERS-wtSpike of SEQ ID NO: 45), or the fragment thereof.

Preferably, the substitution of the amino acid residue at the position
- L506 is selected from L506R;
- G538 is selected G538E, G538K; G538L, G538Y, G538Q, G538T, G538R, or G538D,
- V555 is selected from V555W, V555Y, V555R, V555F.

More preferably, the substitution of the amino acid residue at the position
- L506 is L506R;
- G538 is selected from G538E or G538K,
- V555 is selected from V555W, V555Y, V555R, V555F

More preferably, the MERS-mRBD or the fragment thereof comprises an amino acid sequence or a fragment thereof comprising one or more substitutions of amino acid residues selected from, L506R, G538E, G538K, V555W, V555Y, V555R, V555F.

More preferably, the substitution of the amino acid residue at the position
- L506 is L506R,
- G538 is G538K
- V538 is V555Y.

More preferably, the MERS-mSpike or the fragment thereof comprises an amino acid sequence or a fragment thereof comprising one or more substitutions of amino acid residues selected from L506R, G538K, V555Y. Most preferred is V555Y.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 57 , SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92; SEQ ID NO: 93, SEQ ID NO: 94, SEQ ID NO: 95, SEQ ID NO: 96, SEQ ID NO: 97, SEQ ID NO: 98, SEQ ID NO: 99; or SEQ ID NO: 100, provided that the MERS-mSpike or the fragment thereof has a reduced binding strength to DPP4 as defined above. Preferably, the MERS-mSpike or the fragment thereof further exhibits a binding to anti-MERS-wtRBD neutralizing antibodies as defined above.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from, SEQ ID NO: 58, SEQ ID NO: 59, SEQ ID NO: 60, SEQ ID NO: 61, SEQ ID NO: 62, SEQ ID NO: 63, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92; SEQ ID NO: 93.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92; SEQ ID NO: 93.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 66, SEQ ID NO: 72, SEQ ID NO: 73, SEQ ID NO: 89, SEQ ID NO: 90, SEQ ID NO: 91, SEQ ID NO: 92; SEQ ID NO: 93.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 66, SEQ ID NO: 73, SEQ ID NO: 90.

More preferably, the MERS- mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected of SEQ ID NO: 90. Hence, most preferably, the MERS-mSpike or the fragment thereof comprises, preferably consists of, an amino acid selected of SEQ ID NO: 90.

### Mutant spike protein of MERS-CoV (MERS-mSpike) combining mutations described in (A) and (B)

Preferably, the MERS-mSpike or a fragment thereof according to the invention and as further described in (B) may further comprise the mutations in the MERS-mRBD or the fragment thereof according to the invention as further described in (A).

Hence, the present invention preferably relates to a MERS-mSpike or a fragment thereof according to the present invention as further described above in (B) comprising the MERS-mRBD or the fragment thereof according to the present invention as further described above in (A). Hence, it is to be understood that any embodiment disclosed for the MERS-mRBD or the fragment thereof and in particular under (A) and any embodiment disclosed for the MERS-mSpike or the fragment thereof and in particular under (B) is *mutatis mutandis* applicable to the mSpike comprising both mutations for the MERS-mSpike and mutations for the MERS-mRBD or the fragment thereof.

In the following the substitutions in the mSpike are disclosed as the amino acid positions of the MERS-wtSpike. Reference is again made to table A showing a selection of corresponding amino acid positions in the MERS-wtRBD/MERS-mRBD and the MERS-wtSpike/MERS-mSpike.

Preferably, the one or more substitutions of amino acids in the MERS-mRBD described in (A) may be combined with any of the MERS-mSpike substitutions of amino acids described in (B).

Hence, preferably, any of the one or more substitutions of amino acid residues in the MERS-mRBD as described in (A) may be combined with one or more substitutions of amino acid residues at positions selected from F39, S133, R307, H91 and/or with one or more substitutions of amino acid residues enabling an oligomerization of two or more mSpikes, and/or enabling one or more disulfide bonds, preferably the combination of three substitutions of amino acid residues at positions V458, V1060 and L1061 (VPP) and/or the combination of two substitutions at positions T424 and S459 (TS) of the MERS-wtSpike of SEQ ID NO: 46 or the fragment thereof as described in (B).

Hence, preferably, any of the one or more substitutions of amino acids in the MERS-mSpike or the fragment at positions selected from F39, S133, R307, H91 and/or one or more substitutions of amino acid residues enabling an oligomerization of two or more mSpikes, and/or enabling one or more disulfide bonds, preferably the combination of three substitutions of amino acid residues at positions V458, V1060 and L1061 (VPP) and/or the combination of two substitutions at positions T424 and S459 (TS) of the MERS-wtSpike of SEQ ID NO: 46 may be combined with any of the one or more substitutions of amino acid residues at positions selected from S94, G96, S132, R138, S139, L140, T146, E147, V168, G172, Y174, R176, W187, V189, A190, S191, or S193 of the MERS-wtRBD of SEQ ID NO: 1

Hence, preferably the MERS-mSpike or the fragments thereof comprises an amino acid sequence or a fragment thereof comprising one or more substitutions of amino acid residues at positions selected from S460, G462, S498, K502, S504, R505, L506, T512, E513, V534, G538, Y540, R542, W553, V555, A556, S557, or S559 of the MERS-wtSpike of SEQ ID NO: 46 or the fragment thereof, and one or more substitutions of amino acid residues at positions selected from F39, S133, R307, H91 and/or one or more substitutions of amino acid residues enabling an oligomerization of two or more mSpikes, and/or enabling one or more disulfide bonds, preferably a combination of three substitutions of amino acid residues at positions V458, V1060 and L1061 (VPP) and/or a combination of two substitutions at positions T424 and S459 (TS) of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Preferably, the one or more substitutions of amino acid residues comprised in the amino acid sequence of the MERS-mSpike comprises two to eleven substitutions, more preferably three or ten substitutions, more preferably four substitutions.

Hence, preferably, the one or more substitutions of amino acids in the MERS-mSpike or the fragment comprise, more preferably consisting of, two to eleven, preferably three to ten, more preferably four substitutions of amino acids. Accordingly, the MERS-mSpike according to the invention comprising the MERS-mRBD according to the invention, comprises, more preferably consists of two to eleven, preferably three to ten, more preferably four to five substitutions of amino acids.

Preferably, the one or more substitutions of amino acids in the MERS-mSpike or the fragment thereof at positions selected from F39, S133, R307, H91 comprise, more preferably consisting of, one to three, or two, preferably one or two, more preferably one substitutions of amino acids.

Preferably, the one or more substitutions of amino acids in the MERS-mSpike or the fragment thereof at positions selected from S460, G462, S498, K502, S504, R505, L506, T512, E513, V534, G538, Y540, R542, W553, V555, A556, S557, or S559 comprise, more preferably consisting of, one to three, or two, preferably one or two, more preferably one substitutions of amino acids.

In a preferred embodiment, the MERS-mSpike or the fragments thereof comprises an amino acid sequence or a fragment thereof comprising one or more substitutions of amino acid residues at positions selected from S460, G462, S498, K502, S504, R505, L506, T512, E513, V534, G538, Y540, R542, W553, V555, A556, S557, or S559 of the MERS-wtSpike of SEQ ID NO: 46 or the fragment thereof, and one or more substitutions of amino acid residues at positions selected from F39, S133, R307, H91 of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

More preferably, the MERS-mSpike or the fragments thereof comprises an amino acid sequence or a fragment thereof comprising a substitution of amino acid residues at positions selected from V555 of the MERS-wtSpike of SEQ ID NO: 46 or the fragment thereof, and one or more substitutions of amino acid residues at positions selected from F39, S133, R307, H91 of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Preferably, F39 is F39A, S133 is S133A, R307 is R307A, H91 is H91A.

Preferably, V555 is selected from V555W, V555Y, V555F, V555R V555K, V555A, preferably from V555W, V555Y, V555F, V555R, more preferably V555 is V555Y.

More preferably, the MERS-mSpike or the fragments thereof comprises an amino acid sequence or a fragment thereof comprising a substitution of amino acid residues at positions selected from V555Y and F39A, or V555Y and S133A, or V555A and R307A, or V555A and H91A of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

In a further preferred embodiment, the MERS-mSpike or the fragments thereof comprises an amino acid sequence or a fragment thereof comprising one or more substitutions of amino acid residues at positions selected from S460, G462, S498, K502, S504, R505, L506, T512, E513, V534, G538, Y540, R542, W553, V555, A556, S557, or S559 of the MERS-wtSpike of SEQ ID NO: 46 or the fragment thereof, and one or more substitutions of amino acid residues enabling an oligomerization of two or more mSpikes, and/or enabling one or more disulfide bonds, wherein the MERS-mSpike or the fragment thereof has, except for the substitutions, an amino acid sequence identity of 80% or more to SEQ ID NO: 46.

Preferably, one or more substitutions of amino acid residues enabling an oligomerization of two or more mSpikes, and/or enabling one or more disulfide bonds is a combination of three substitutions of amino acid residues at positions V458, V1060 and L1061 (VPP), more preferably a combination of two substitutions at positions T424 and S459 (TS) of the MERS-wtSpike of SEQ ID NO: 46.

More preferably, the MERS-mSpike or the fragments thereof comprises an amino acid sequence or a fragment thereof comprising a substitution of amino acid residues at positions selected from G538 of the MERS-wtSpike of SEQ ID NO: 46 or the fragment thereof, and one or more substitutions of amino acid residues enabling an oligomerization of two or more mSpikes, combination of two substitutions at positions T424 and S459 (TS) of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46

Preferably, G538 is selected from G538E, G538K or G538Q

Preferably, T424 is T424C and S459 is S459C.

More preferably, the MERS-mSpike or the fragments thereof comprises an amino acid sequence or a fragment thereof comprising a substitution of amino acid residues at positions selected from G538E and T424C and S459C, or G538K and T424C and S459C, or G538Q and T424C and S459C of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

Still more preferably, the MERS-mSpike or the fragment thereof comprises, preferably consists of, an amino acid sequence having a sequence identity of 85% or more, preferably 90% or more, more preferably of 95% or more, still more preferably of 99% or more, most preferably of 100% to an amino acid sequence selected from SEQ ID NO: 147, SEQ ID NO: 148, SEQ ID NO: 149, SEQ ID NO: 150, SEQ ID NO: 151, SEQ ID NO: 152, SEQ ID NO: 153.

### Polypeptides and proteins comprising the MERS-mRBD or the MERS-mSpike

As stated above, the present invention preferably relates to a polypeptide or protein comprising the MERS-mRBD or the fragment thereof according to the present invention or the MERS-mSpike or the fragment thereof according to the present invention in any one of the embodiments described above.

Preferably, the polypeptide and/or protein comprising the MERS-mRBD or the fragment thereof or the MERS-mSpike or the fragment thereof is a recombinant polypeptide or protein, or a chemically synthesized polypeptide or protein.

The polypeptide or protein may comprise one or more additional amino acid sequences other than the MERS-mRBD or the fragment thereof or the MERS-mSpike or the fragment thereof. Such additional amino acid sequences may be a domain or fragment of a protein different to the MERS-mRBD or the fragment thereof and/or the MERS-spike or the fragment thereof, a localisation signal like a cytosol localisation signal or a secreting signal, a stability-regulating amino acid sequence like sequences promoting or preventing the active and/or passive degradation of the polypeptide or protein, polymerizsation domains, like di- or trimerisation domains, a spacer, a linker, a tag like a FLAG-tag or His-tag, etc. Furthermore, the polypeptide or protein may comprise one or more modifications like a glycosylation.

Furthermore, in the present invention, it is to be understood that any embodiment disclosed for the MERS-mRBD or the fragment thereof and/or for the MERS-mSpike or the fragment thereof is/are mutatis mutandis applicable to the polypeptide or protein comprising the MERS-mRBD or the fragment thereof or MERS-mSpike or the fragment thereof.

### Nucleic acid

Furthermore, the present invention preferably relates to a nucleic acid comprising, preferably consisting of, a nucleotide sequence encoding for:
- the MERS-mRBD or the fragment thereof according to the present invention;
- the MERS-mSpike or the fragment thereof according to the present invention; or
- the polypeptide or protein according to the present invention comprising the MERS-mRBD or the fragment thereof according to the present invention or the MERS-mSpike or the fragment thereof according to the present invention.

Preferably, the nucleic acid is a DNA or RNA like a mRNA.

Preferably, the nucleic acid is a recombinant nucleic acid or a chemically synthesized nucleic acid.

Furthermore, the nucleic acid may comprise one or more additional sequences other than the nucleotide sequence encoding for the MERS-mRBD or the fragment thereof, the MERS-mSpike or the fragment thereof or the polypeptide or protein according to the present invention. Such additional nucleotide sequences may be a transcription regulatory sequence like a promoter, an enhancer, terminator, a transcription factor binding motif or a DNA-polymerase or reverse transcriptase binding site, a translation regulatory sequence like a ribosome binding motif, an exon, an intron, a 5'-cap, a poly-A-tail, a localisation signal like a core or cytosol localisation signal, hybridisation stretches and/or a stability regulating sequence nucleotide sequences promoting or preventing the active and/or passive degradation of the nucleic acid, spacer, linker, sequences encoding a tag like a FLAG-tag or His-tag etc. Furthermore, the nucleic acid may comprise modifications like methylation.

Furthermore, in the present invention, it is to be understood that any embodiment disclosed for:
the MERS-mRBD or the fragment thereof; and/or
the MERS-mSpike or the fragment thereof, and/or
the polypeptide or protein comprising the MERS-mRBD or the fragment thereof or the MERS-mSpike or the fragment thereof,
is/are mutatis mutandis applicable to the nucleic acid according to the present invention.

### Vaccine composition and Medical use

The present invention as disclosed in any one of the embodiments described herein may preferably be for use as a vaccine.

Accordingly, the present invention preferably relates to:
- one or more MERS-mRBDs or the fragments thereof according to the present invention;
- one or more MERS-mSpikes or the fragments according to the present invention;
- one or more polypeptides or proteins according to the present invention comprising the MERS-mRBD or the fragment thereof according to the present invention or the MERS-mSpike or the fragment thereof according to the present invention; and/or
- one or more nucleic acids according to the present invention comprising a nucleotide sequence encoding for the one or more MERS-mRBDs or the fragments thereof, the one or more MERS-mSpikes or the fragments thereof or the one or more polypeptides or proteins; and/or
- the vaccine composition as described above in view of the MERS-mRBD and the MERS-mSpike,
for use as a vaccine.

Furthermore, preferably, the present invention relates to a vaccine composition comprising, preferably consisting of, as an active ingredient:
- one or more MERS-mRBDs or the fragment thereof according to the present invention;
- one or more MERS-mSpikes or the fragments thereof according to the present invention;
- one or more polypeptides or proteins according to the present invention comprising the MERS-mRBD or the fragment thereof according to the present invention or the MERS-mSpike or the fragment thereof according to the present invention; and/or
- one or more nucleic acids according to the present invention comprising a nucleotide sequence encoding for the one or more MERS-mRBDs or the fragments thereof, the one or more MERS-mSpikes or the fragments thereof or the one or more polypeptides or proteins.

Preferably, the vaccine composition further comprises a pharmaceutically-acceptable adjuvant, carrier, diluent and/or excipient.

Preferably, the vaccine composition comprises the active ingredient in an effective amount.

Preferably, the vaccine composition is capable of inducing an immune response against MERS-CoV.

Preferably, the vaccine composition is a vaccine for the prevention and/or treatment of MERS caused by MERS-CoV.

Preferably, the vaccine composition is for use in the prevention and/or treatment of MERS caused by MERS-CoV.

Furthermore, the present invention preferably relates to:
- one or more MERS-mRBDs or the fragments thereof according to the present invention;
- one or more MERS-mSpikes or the fragments according to the present invention;
- one or more polypeptides or proteins according to the present invention comprising the MERS-mRBD or the fragment thereof according to the present invention or the MERS-mSpike or the fragment thereof according to the present invention; and/or
- one or more nucleic acids according to the present invention comprising a nucleotide sequence encoding for the one or more MERS-mRBDs or the fragments thereof, the one or more MERS-mSpikes or the fragments thereof or the one or more polypeptides or proteins; and/or
- the vaccine composition as described above in view of the MERS-mRBD and the MERS-mSpike,
for use in the prevention and/or treatment of a disease, preferably MERS caused by MERS-CoV in a subject.

Preferably, in the vaccine composition and/or the uses in the prevention and/or treatment, one or more is two or more, more preferably one to five, even more preferably two to four and still more preferably two. Preferably, in case e.g. two or more MERS-mRBDs are present, these differ in their sequence to each other, for instance due to the presence of different substitutions. The same applies to the presence of two, three or more MERS-mSpikes, polypeptides or proteins and/or nucleic acids.

Preferably, the term subject as used for the vaccine composition and/or the use in the prevention and/or treatment is a vertebrate, more preferably a mammal, more preferably an camel, dromedary camel, ape, monkey, lemur, canine like a dog, wolf or fox, a cat like a big or small cat, a rodent like a mouse, rat, guinea pig, hamster or rabbit, a bovid like a buffalo, antelope, sheep, goat or cattle, a deer, a horse, a donkey, a bear, marten, bat and/or human, still more preferably the subject is a human or a camel, dromedary camel. The vaccination of farm animals such as camels or dromedary camels, that carry MERS-CoV may be a good alternative to vaccinating humans, as MERS-CoV spills over from dromedary camels to human. Vaccinating farm animals thus might prevent the spill over.

Preferably, in the vaccine composition and/or the use in the prevention and/or treatment, the subject is non-infected with the MERS-CoV and/or is infected with the MERS-CoV. The subject non-infected with the MERS-CoV may previously had or had not passed through a MERS-CoV-infection. The subject infected with the MERS-CoV may presently has a MERS-CoV infection. For determining whether a subject is non-infected with the MERS-CoV or is infected with the MERS-CoV, any method known to the person skilled in the can be used. An example for a suitable method is a PCR-test or a serological test for the presence of MERS-CoV specific antibodies.

Preferably, the MERS-CoV as used in the present invention includes any variants thereof, like AF88936.1, AFY13307.1, AGG22542.1, AGV08379.1, AGV08584.1, AHI48528.1, AHI48733.1, AHC74088.1, AGV08438.1, AID55090.1, AID55095.1, AID55087.1, AKL59401.1, ALB08322.1, ALB08289.1, AHY22545.1, AHL18090.1, AHX00711.1 , AHX0072.1 and AHY22555.1.

More preferably, the one or more MERS-mRBDs, MERS-mSpike or the fragments thereof is any one or any combination of the MERS-mRBDs and/or MERS-mSpike in any of the embodiments as described herein. Preferably, in case the vaccine comprises two or more MERS-mRBDs, MERS-mSpike or the fragments thereof the two or more MERS-mRBDs or the fragments thereof preferably differ in their substitution.

Hence, preferably, the one or more MERS-mRBD, MERS-mSpike or fragments thereof is a combination of two or more MERS-mRBD, MERS-mSpike or fragments thereof, comprising each an amino acid sequence or a fragment thereof comprising one or more substitutions as defined in any of the embodiments as described herein. Preferably, the two or more MERS-mRBD, MERS-mSpike or fragments thereof differ in their substitution. Thus, preferably one MERS-mRBD, MERS-mSpike or fragments thereof comprises, preferably consists of, an amino acid sequence or a fragment thereof comprising one or more substitutions and the second MERS-mRBD, MERS-mSpike or fragments thereof comprises, preferably consists of, an amino acid sequence or a fragment thereof comprising one or more substitutions which are different.

Hence, combinations of two or more, preferably two, different RBD mutants or two or more, preferably two, different Spike mutants as described in any of the embodiments as disclosed herein may be used in the vaccine composition and/or the use in the prevention and/or treatment according to the invention. In other words, the vaccine composition and/or the use preferably comprises a combination of two different MERS-mRBDs or two different MERS-mSpikes as described in any of the embodiments as disclosed herein. Particularly preferred combinations are substitutions of the amino acid residues at positions L506 and G462, or L506 and G538, or G538 and V555, G538 and G462, or L506 and V555 of the MERS-wtSpike of SEQ ID NO: 46.

Hence, preferably, the vaccine composition comprises a MERS-mRBD or MERS-mSpike or a fragment thereof comprising, or preferably consisting of, an amino acid sequence or a fragment thereof comprising a substitution of amino acid residues at position L506, preferably L506R, and a further MERS-mRBD or MERS-mSpike or a fragment thereof comprising, or preferably consisting of, an amino acid sequence or a fragment thereof comprising a substitutions of amino acid residues at position G462, preferably G462L of the MERS-wtSpike of SEQ ID NO: 46.

Furthermore, preferably the vaccine composition comprises a MERS-mRBD or MERS-mSpike or a fragment thereof comprising, or preferably consisting of, an amino acid sequence or a fragment thereof comprising a substitutions of amino acid residues at position L506, preferably L506R, and a further MERS-mRBD or MERS-mSpike or a fragment thereof comprising, or preferably consisting of, an amino acid sequence or a fragment thereof comprising a substitutions of amino acid residues at position G538, preferably G538E or G538K of the MERS-wtSpike of SEQ ID NO: 46.

Furthermore, preferably the vaccine composition comprises a MERS-mRBD or MERS-mSpike or a fragment thereof comprising, or preferably consisting of, an amino acid sequence or a fragment thereof comprising a substitutions of amino acid residues at position G538, preferably G538E or G538K, and a further MERS-mRBD or MERS-mSpike or a fragment thereof comprising, or preferably consisting of, an amino acid sequence or a fragment thereof comprising a substitutions of amino acid residues at position V555, preferably V555Y or V555W of the MERS-wtSpike of SEQ ID NO: 46.

Furthermore, preferably the vaccine composition comprises a MERS-mRBD or MERS-mSpike or a fragment thereof comprising, or preferably consisting of, an amino acid sequence or a fragment thereof comprising a substitutions of amino acid residues at position G538, preferably G538E or G538K, and a further MERS-mRBD or MERS-mSpike or a fragment thereof comprising, or preferably consisting of, an amino acid sequence or a fragment thereof comprising a substitutions of amino acid residues at position G462, preferably G462L of the MERS-wtSpike of SEQ ID NO: 46.

Furthermore, preferably the vaccine composition comprises a MERS-mRBD or MERS-mSpike or a fragment thereof comprising, or preferably consisting of, an amino acid sequence or a fragment thereof comprising a substitutions of amino acid residues at position L506, preferably L506R, and a further MERS-mRBD or MERS-mSpike or a fragment thereof comprising, or preferably consisting of, an amino acid sequence or a fragment thereof comprising a substitution of amino acid residues at position V555, preferably V555Y or V555W of the MERS-wtSpike of SEQ ID NO: 46.

Furthermore, preferably, the vaccine composition comprises a MERS-mRBD or MERS-mSpike or a fragment thereof comprising, or preferably consisting, of an amino acid sequence having a sequence identity of 85% or more to an amino acid sequence selected from SEQ ID NO: 66 and a further MERS-mRBD or MERS-mSpike or a fragment thereof comprising, or preferably consisting, of an amino acid sequence having a sequence identity of 85% or more to an amino acid sequence selected from SEQ ID NO: 66.

Furthermore, preferably, the vaccine composition comprises a MERS-mRBD or MERS-mSpike or a fragment thereof comprising, or preferably consisting, of an amino acid sequence having a sequence identity of 85% or more to an amino acid sequence selected from SEQ ID NO: 66 and a further MERS-mRBD or MERS-mSpike or a fragment thereof comprising, or preferably consisting, of an amino acid sequence having a sequence identity of 85% or more to an amino acid sequence selected from SEQ ID NO: 72 or SEQ ID NO: 73.

Furthermore, preferably, the vaccine composition comprises a MERS-mRBD or MERS-mSpike or a fragment thereof comprising, or preferably consisting, of an amino acid sequence having a sequence identity of 85% or more to an amino acid sequence selected from SEQ ID NO: 72 or SEQ ID NO: 73 and a further MERS-mRBD or MERS-mSpike or a fragment thereof comprising, or preferably consisting, of an amino acid sequence having a sequence identity of 85% or more to an amino acid sequence selected from SEQ ID NO: 89 or SEQ ID NO: 90.

Furthermore, preferably, the vaccine composition comprises a MERS-mRBD or MERS-mSpike or a fragment thereof comprising, or preferably consisting, of an amino acid sequence having a sequence identity of 85% or more to an amino acid sequence selected from SEQ ID NO: 72 or SEQ ID NO: 73 and a further MERS-mRBD or MERS-mSpike or a fragment thereof comprising, or preferably consisting, of an amino acid sequence having a sequence identity of 85% or more to an amino acid sequence selected from SEQ ID NO: 60.

Furthermore, preferably, the vaccine composition comprises a MERS-mRBD or MERS-mSpike or a fragment thereof comprising, or preferably consisting, of an amino acid sequence having a sequence identity of 85% or more to an amino acid sequence selected from SEQ ID NO: 66 and a further MERS-mRBD or MERS-mSpike or a fragment thereof comprising, or preferably consisting, of an amino acid sequence having a sequence identity of 85% or more to an amino acid sequence selected from SEQ ID NO: 89 or SEQ ID NO: 90.

Furthermore, a vaccine composition may preferably be administered one or more times, preferably one to three four, more preferably two to three times. Hence, preferably one or more immunizations may be conducted, such as prime and boost immunizations. The first, second and further immunization may be conducted both with MERS-mRBD or MERS-mSpike or with mixtures of MERS-mRBD and MERS-mSpike. For example, preferably, the first immunization may be conducted with a full MERS-mSpike and the second immunization may be conducted with a MERS-mRBD or vice versa.

Furthermore, the first, second and further immunization may be conducted with similar MERS-RBD or MERS-Spike mutants or with different MERS-RBD or MERS-Spike mutants as described in any of the embodiments as disclosed herein. In other words, the MERS-mRBDs or MERS-mSpike used for the first immunization may may have similar or different substitutions as the MERS-mRBDs or MERS-mSpike used for the second and further immunizations.

Furthermore, preferably the first and second immunization may be conducted with different MERS-mRBD or MERS-mSpike as described in any of the embodiments as disclosed herein. Hence, in such preferred embodiment combinations of two or more, preferably two different MERS-mRBDs or MERS-mSpikes are used for vaccination.

For example preferably, the first immunization may be conducted with a MERS-mSpike comprising, more preferably consisting of, an amino acid sequence comprising the substitution V555Y of the MERS-wtSpike of SEQ ID NO: 46 and the second immunization may be conducted with a MERS-mRBD comprising, more preferably consisting of, an amino acid sequence comprising the similar substitution V189Y of the MERS-wtRBD of SEQ ID NO: 1 corresponding to V555Y of the MERS-wtSpike of SEQ ID NO: 46.

For example, preferably the first immunization may be conducted with a MERS-mSpike or fragment thereof comprising, more preferably consisting of, an amino acid sequence comprising the substitutions T424C and TS459C (TS) and the second immunization may be conducted with a MERS-mRBD or MERS-mSpike or fragment thereof comprising, more preferably consisting of, an amino acid sequence comprising the substitution V189Y of the MERS-wtRBD of SEQ ID NO: 1 corresponding to V555Y of the MERS-wtSpike of SEQ ID NO: 46.

More preferably, the one or more nucleic acids according to the present invention comprising a nucleotide sequence encoding for the one or more MERS-mRBDs or the fragments thereof, the one or more MERS-mSpikes or the fragments thereof or the one or more polypeptides or proteins is any one or any combination of the nucleic acids according to the present invention.

Furthermore, in the present invention, it is to be understood that any embodiment disclosed for:
the MERS-mRBD or the fragment thereof; and/or
the MERS-mSpike or the fragment thereof; and/or
the polypeptide or protein comprising the MERS-mRBD or the fragment thereof or the MERS-mSpike; and/or
the nucleic acid nucleic acid according encoding for the same
is/are *mutatis mutandis* applicable to the vaccine composition and/or the use in the prevention and/or treatment.

Furthermore, the present invention relates to a method for the prevention and/or treatment of diseases caused by MERS-CoV, preferably Middle East Respiratory Syndrome in a subject comprising administering to the subject:
- one or more MERS-mRBDs or the fragments thereof according to the present invention;
- one or more MERS-mSpikes or the fragments according to the present invention;
- one or more polypeptides or proteins according to the present invention;
- one or more nucleic acids according to the present invention; and/or
- the vaccine composition according to the present invention.

Moreover, the present invention relates to a method for inducing an immune response, preferably against MERS-CoV in a subject comprising administering to the subject:
- one or more MERS -mRBDs or the fragments thereof according to the present invention;
- one or more MERS -mSpikes or the fragments according to the present invention;
- one or more polypeptides or proteins according to the present invention;
- one or more nucleic acids according to the present invention; and/or
- the vaccine composition according to the present invention.

Furthermore, in the present invention, it is to be understood that any embodiment disclosed for the MERS-mRBD or the fragment thereof and/or for the MERS-mSpike or the fragment thereof, the polypeptide or protein comprising MERS-mRBD or the fragment thereof or the MERS-mSpike, nucleic acid nucleic acid according encoding for the same, the vaccine composition comprising the same, the use in the prevention and/or treatment and/or the method for obtaining the same is/are *mutatis mutandis* applicable to the method for the prevention and/or treatment of diseases caused by MERS-CoV and/or the method for inducing an immune response.

The present invention is further illustrated by the following figures and examples.

### BRIEF DESCRIPTION OF THE DRAWINGS

**Figure 1** shows that Human DPP4 Is highly abundant in human blood. (A), Enzymatic activity of recombinant human DPP4 at indicated concentrations was determined by cleavage of the Gly-Pro-pNA substrate (405nm absorbance after cleavage) and measured at indicated times. (B), Human recombinant DPP4 was added at indicated concentrations to untreated serum (left) and to serum that was depleted from proteins bigger than 50kDa including endogenous DPP4. (C) Concentration of soluble DPP4 in plasma samples of human healthy donors (n=90, one technical replicate, median plus standard deviation).

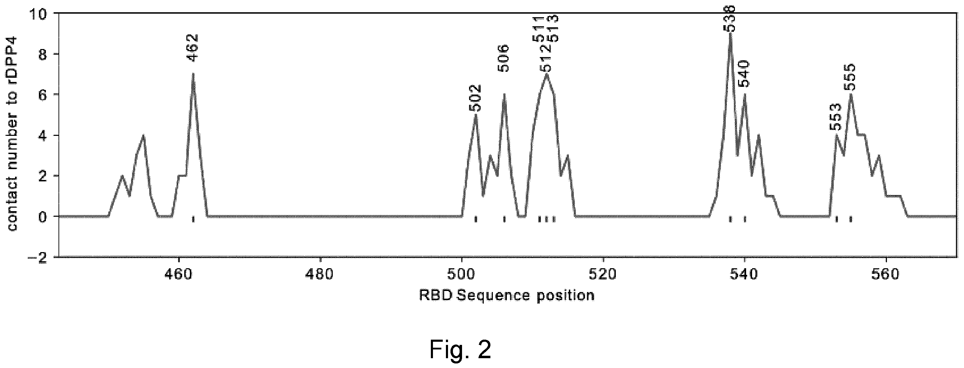

**Figure 2** shows the top 10 MERS-CoV RBD residues serving as contact points for binding to human DPP4. The PDB structure 4L72 was used for a proximity analysis of contact residues likely to be involved in binding to human DPP4.
**Figure 3** shows that disulphide-linkage of MERS-CoV spike proteins abrogates binding to DPP4. Schematic overview of the design of MERS-CoV spike trimer stabilized in the closed conformation by disulphide-linkage of (A) RBD with RBD (T424C S459C (TS)) or (B) RBD with the S2 (V458C V1060K L1061C (VPP)). The spike consists of the S1 head portion, comprising the RBD and the NTD-domain, as well as the S2 stem. (C) Western blot of 200 ng of designed S-protein constructs VPP and TS following reducing and non-reducing SDS-PAGE, using goat anti-His antibodies. (D) BN-PAGE of 2.5 mg of designed S-protein constructs, visualized by Coommassie staining, detected by Li-Cor Odyssey IR scanner. All spike proteins, except VPP, also contain the so-called 2P mutation for stabilization with proline substitutions at residues V1060 and L1061 (J. Pallesen et al.: "Immunogenicity and structures of a rationally designed prefusion MERS-CoV spike antigen", PNAS, August 14, 2017, https://www.pnas.org/content/pnas/114/35/E7348.full.pdf)

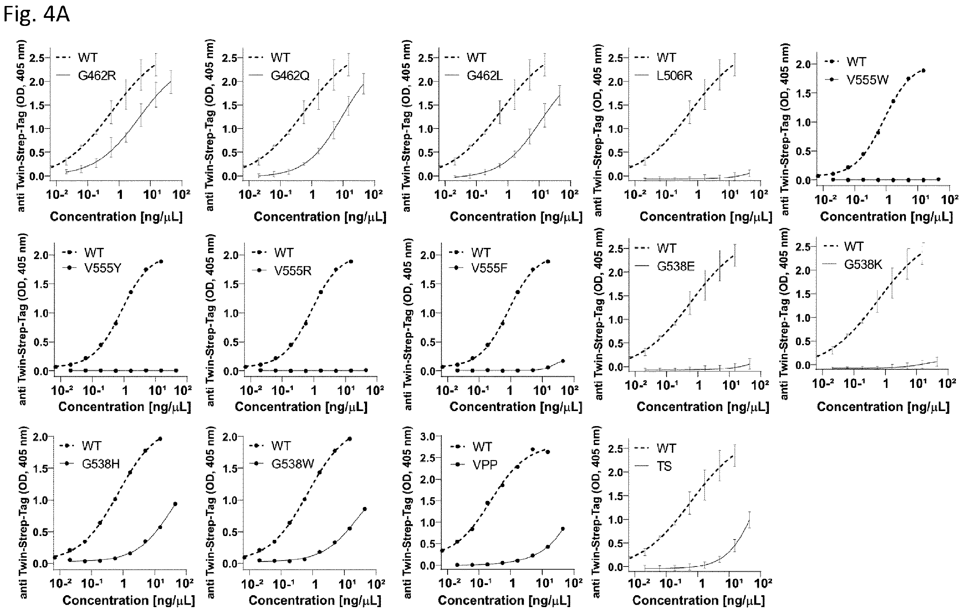

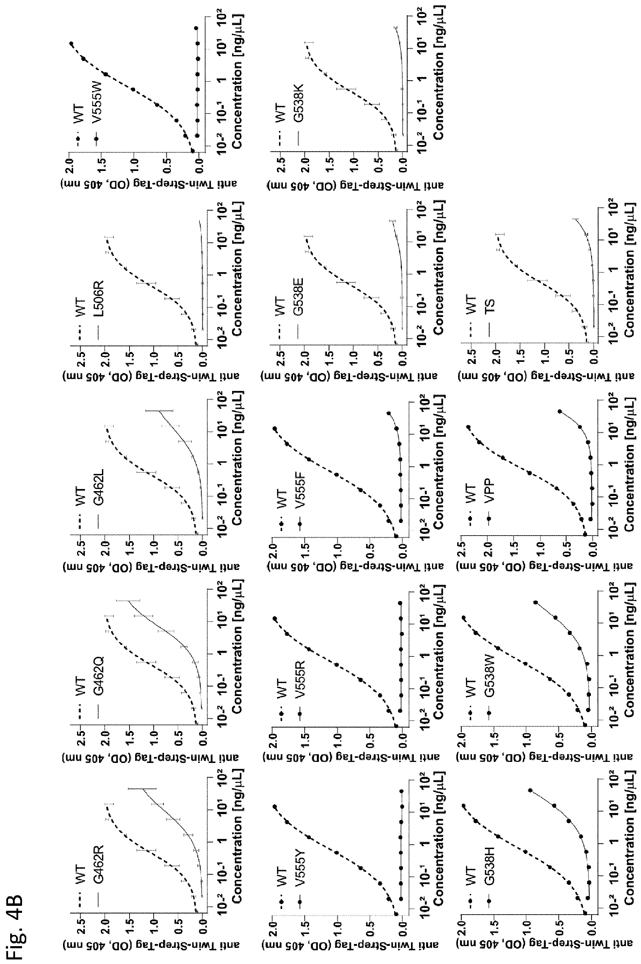

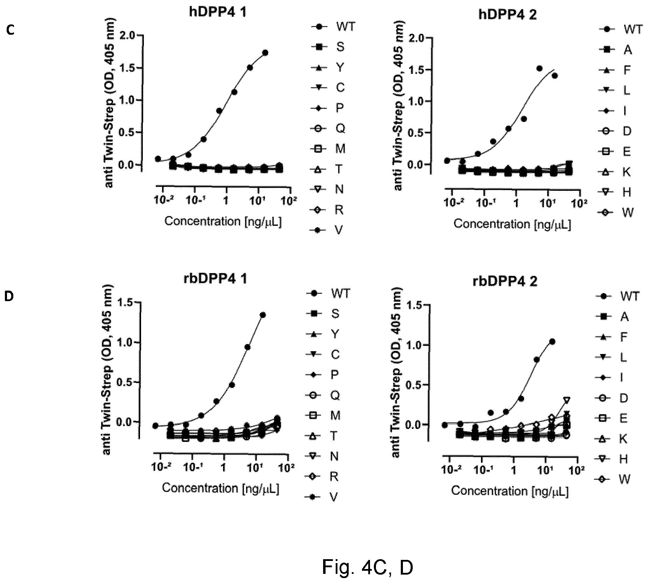

**Figure 4** shows binding of MERS-CoV-RBD variants to human and rabbit DPP4. (A) and (C) MERS-CoV spike-2P (WT) and mutants were tested for binding to human DPP4 and (B) and (D) rabbit DPP4 in ELISA. Binding curves were generated using log(agonist) vs. response - FindECanything in GraphPad Prism. When applicable, means (of n=3 technical replicates) and standard deviations are shown.

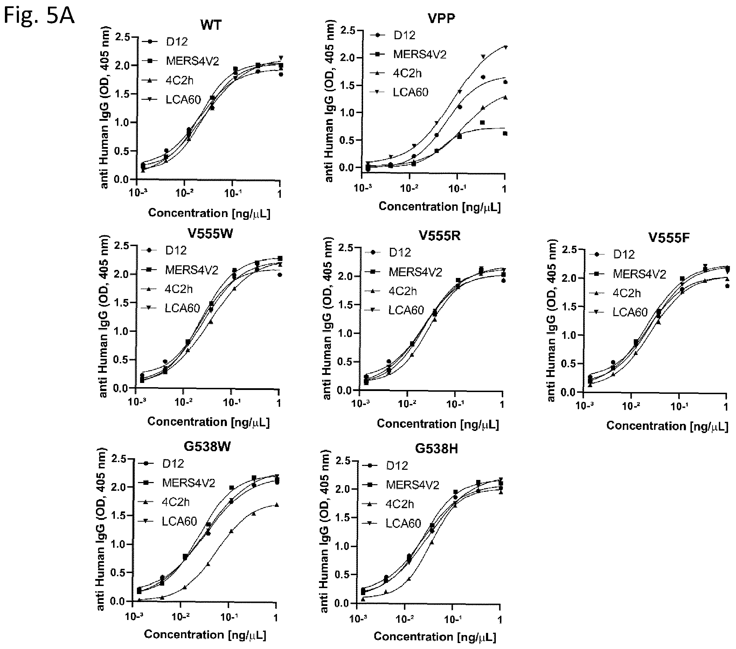

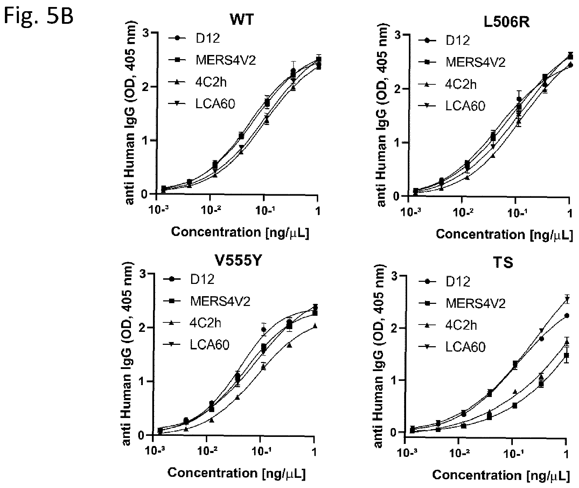

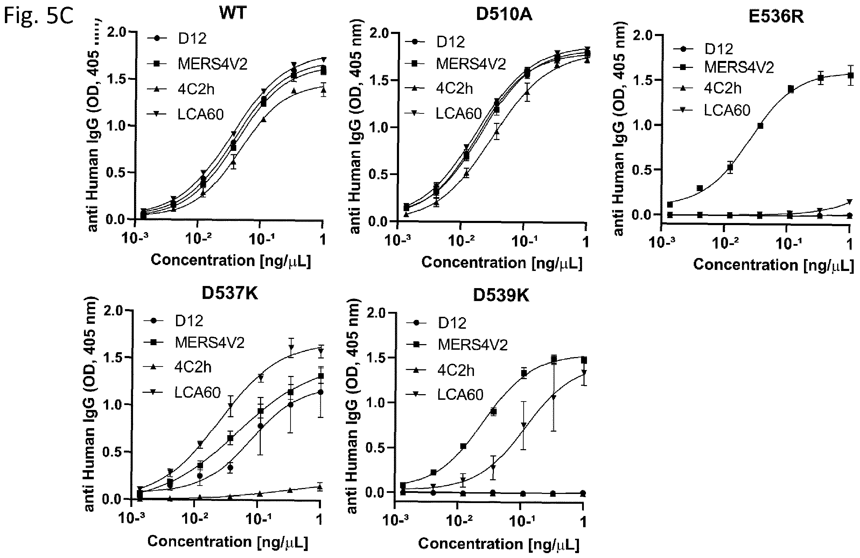

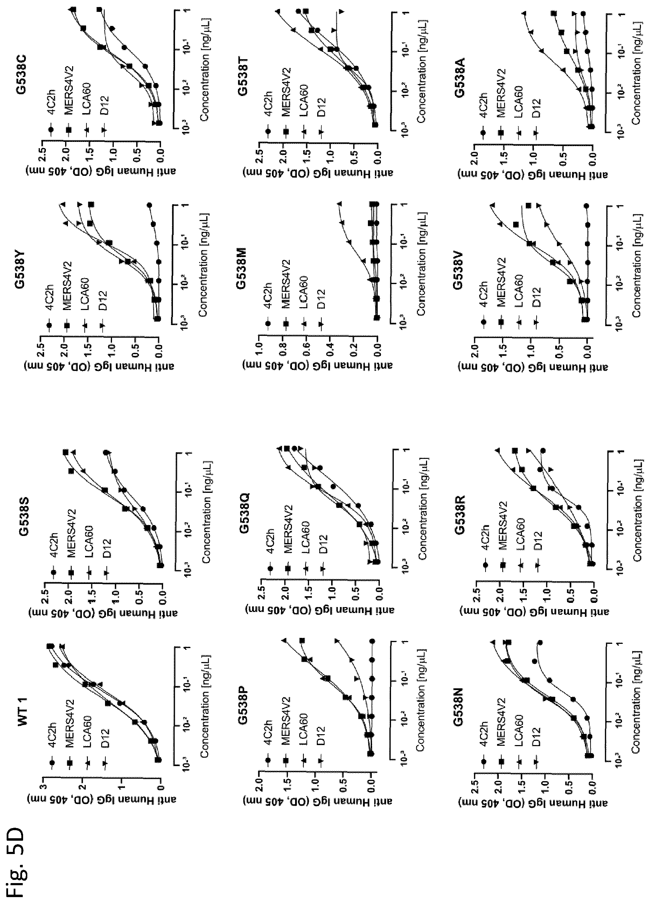

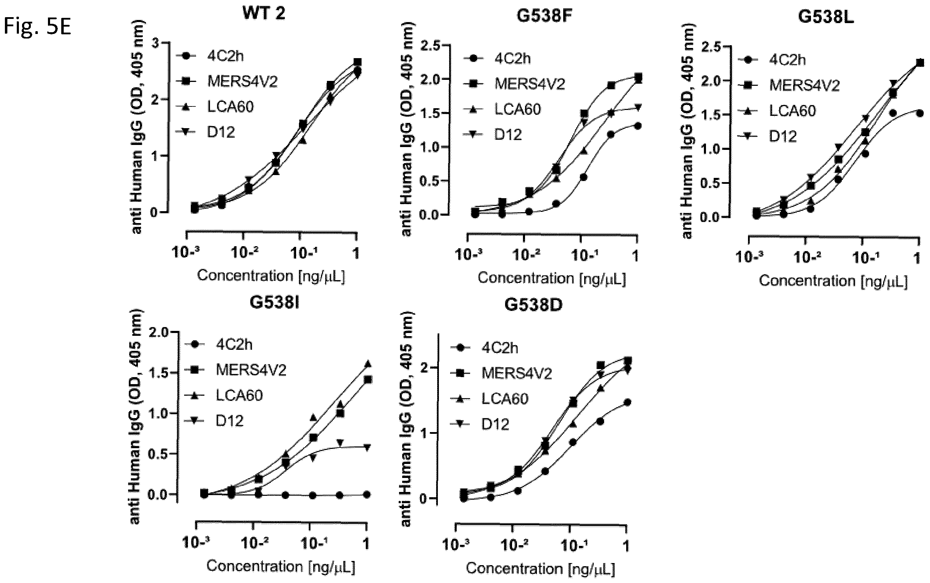

**Figure** 5 shows binding of MERS-CoV S and RBD mutants to selected nAbs. Binding of MERS-CoV specific nAbs D12, MERS4V, 4C2, and LCA60 to indicated MERS-CoV spike mutants compared to wildtype (WT) controls was tested by ELISA in independent experiments using recombinant spike (A,B, D, E) or RBD (C). Mutants tested in (C) have been previously described. If applicable, n=3 technical replicates and standard deviations are shown. Binding curves were generated using log(agonist) vs. response -FindECanything in GraphPad Prism.
**Figure** 6 shows that Human DPP4 has higher binding affinity than rabbit DPP4 affecting epitope masking according to occupancy modelling. (A) Binding of recombinant mouse DPP4 (mDPP4), rabbit DPP4 (rbDPP4) and human DPP4 (hDPP4) to MERS-CoV RBD was determined by ELISA. (B) Concentration of enzymatically active DPP4 in n=8 pre-immune rabbit in 3 technical replicates was detected by cleavage of the Gly-Pro-pNA substrate using standard curves of recombinant rbDPP4. Median and standard deviation is shown. (C) Receptor-ligand binding kinetics of recombinant hDPP4 and recombinant rbDPP4 binding to MERS-CoV RBD determined by Biolayer Interferometry. (D) Models of antigen occupancy of MERS-CoV spike by circulating human versus rabbit DPP4 for Kds of 7.8 nM versus 34.3 nM, respectively. The following assumptions were made: 1 µg MERS-CoV spike per immunization, a tissue distribution volume of 3 ml, availability of one RBD per spike trimer, a molecular weight (MW) of 89 kDa for DPP4 and 630 kDa MW per spike trimer and concentration of human versus rabbit DPP4 of 639 versus 897 ng/ml, respectively. Calculations were based on the formula Kd=[Spike][DPP4]/[Complex]. (E) Modelling the spike occupancy by human DPP4 for vaccine application volumes of 1 ml (actual volume applied in rabbit immunization protocol) versus 30 ml for increasing amounts of immunogen.

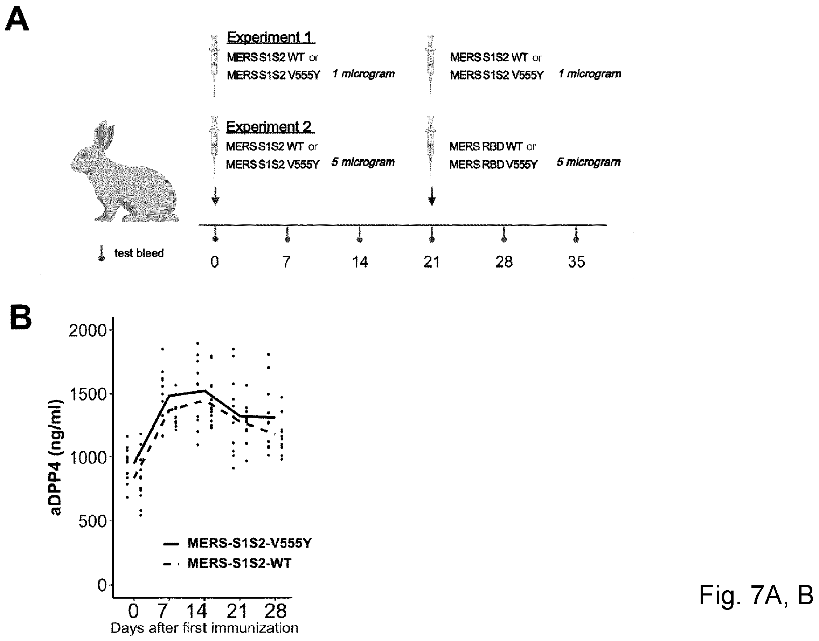

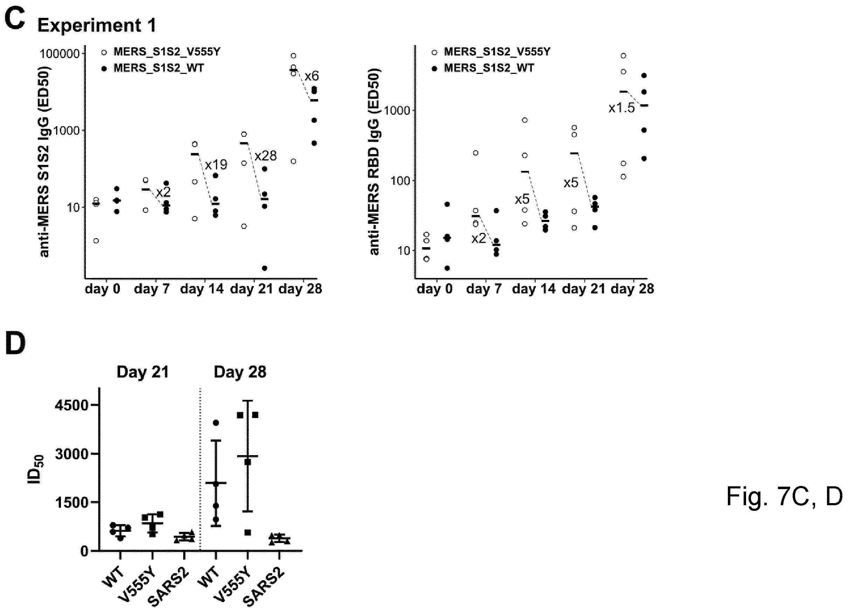

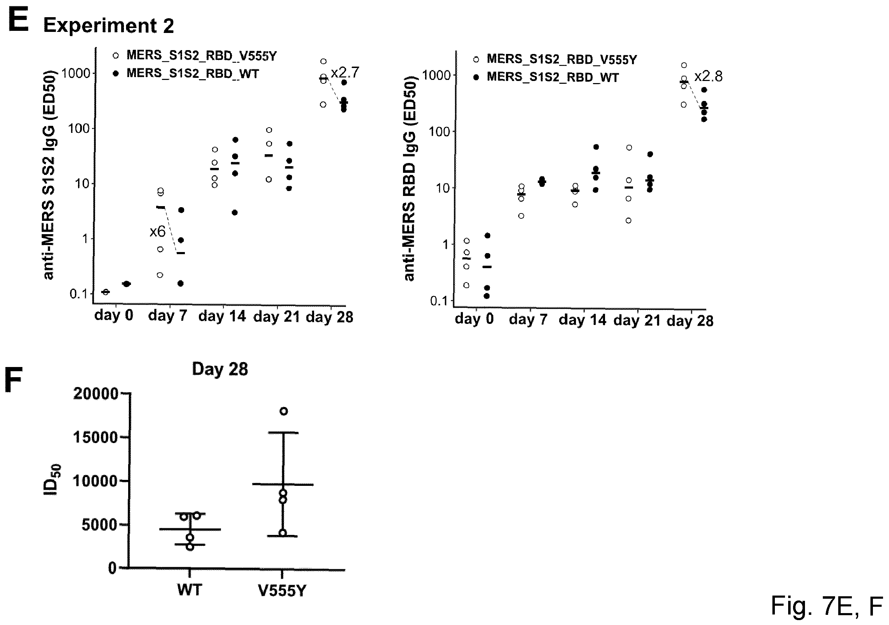

**Figure 7** shows that abrogation of DPP4 binding fosters spike reactive antibodies in mice. (A) Rabbit immunization scheme for Experiment 1 and Experiment 2 depicting i.m. applications of 1 or 5 µg wildtype or mutant spike at day 0 and 21 as well as bleedings conducted at day 0, 7, 14, 21, 28 and 35. (B) Levels of enzymatically active DPP4 were measured at indicated time point post immunization. (C) and (E) MERS-CoV RBD (left) and full spike (right) reactive IgG antibodies were determined for wt versus V555Y immunizations at indicated timepoints after primary immunization. (D) and (F) Neutralization potential of serum antibodies indicated as dilution at which 50% of infection is blocked (inhibitory dilution 50, ID50) was determined by MERS-CoV spike pseudotyped viruses at d21 and d28 after the first immunization.
**Figure 8** shows that MERS-CoV RBD binds to human T cells. (A) Gating of human PBMCs that were stained with a fluorescently labelled MERS-CoV RBD and CD3, CD14, and CD19 specific antibodies to discriminate T cells, monocytes and B cells, respectively. (B) Percent RBD positive cells were determined in 4 independent donors.

### EXAMPLES

### Methods

### 1. Cloning:

In the DPP4 and monoclonal antibody constructs, protein N-termini were preceded by an IgH signal peptide (SP) sequence, with the sequence MGWSCIILFLVATATGVHS (SEQ ID No: 101), to facilitate protein secretion. Non-antibody proteins included either an 8xHis-tag or a 6xHis-tag on the N- or C-terminus for purification purposes. Gibson Assembly cloning was performed using the Gibson Assembly Master Mix (New England Biolabs, #E2611) according to the manufacturer's instructions. Chemically competent E. coli for transformation of plasmids were produced in-house and transformed conforming to the NEB transformation protocol (New England Biolabs, #E1601). Isolation of plasmids from bacterial cultures was done using the PureYieldTM Plasmid Miniprep System (Promega, #A1222) and PureYieldTM Plasmid Midiprep System (Promega, #A2496) according to the manufacturer's instructions. The sequences of the expression cassettes were verified by DNA sequencing (LGC Genomics GmbH).

### a. DPP4 constructs

The gene for recombinant human DPP4 with an N-terminal 8xHis-tag joint to the protein by a 3xGGGS linker (hDPP4-N'His) containing the S38-P766 fragment of human DPP4 (Uniprot, P27487) was synthesized by GenScript and cloned into a mammalian expression vector containing an SP using Gibson Assembly.

The gene for recombinant rabbit DPP4 with an C-terminal 6xHis-tag (rbDPP4-His) was obtained by GenScript (Clone ID:OOb07969C).

### b. MERS-CoV constructs

The MERS-CoV RBD amino acids G372-L588 were cloned in front of a GGGGS-linker, upstream of a Twin Strep tag sequence followed by a C-terminal 6xHis-tag (GGGGSWSHPQFEKGGGSGGGSGGSAWSHPQFEKHHHHHH, SEQ ID: 103). This was introduced into the mammalian expression vector pcDNA3.1+ containing the signal peptide spanning amino acids M1-Q14 of the Wuhan SARS-CoV-2 isolate (GenBank: MN908947.3) an AviTag^{™} and a GG linker (MFVFLVLLPLVSSQGLNDIFEAQKIEWHEGG, SEQ ID NO: 102).

The MERS-CoV Spike amino acids 1-1291 were cloned in front of a TEV protease site, foldontrimerization motif, Twin Strep tag sequence, 6xHis-tag (SEQ ID NO: 104: SGRENLYFQGGGGSGYIPEAPRDGQAYVRKDGEWVLLSTFLGWSHPQFEKGGGSGGGSGGSAWSHP QFEKHHHHHH). This was introduced into the mammalian expression vector pcDNA3.1+. The MERS-CoV S1/S2 furin cleavage site was mutated and MERS-CoV Spike were stabilized with the 2P mutations (Pallesen et al., 2017).

The amino acid modifications were introduced by PCR mutagenesis to create the RBD-mutants and Spike-mutants with abrogated DPP4 binding using the Q5 Site-Directed Mutagenesis Kit (New England Biolabs, #E0554) according to the manufacturer's instructions.

### c. Monoclonal antibody constructs

Synthetic V(D)J inserts of monoclonal antibodies LCA60, 4C2h, D12 and MERS-4V2 were individually generated by overlapping PCR (see table 1-2). PCR products were recovered using ProNex beads (Promega, #NG2002) in agreement with the manufacturer's instructions. Subsequently, using Gibson Assembly, the PCR products were cloned into mammalian expression vectors containing an SP and the Ig kappa constant region R1-C107 (light chain) or the IgG1 H constant region A1-K330 (heavy chain).

**Table 1**

| **Antibody** | **Primer** | **Chain** | **Sequence (5'-3')** | **Seq. ID NO:** |
|---|---|---|---|---|
| LCA60 | F1 | Heavy | CTGCAACCGGTGTACATTCTGAGGTGCAGCTG CTGGAGAGCGGCGGCGGCCTGGTGAAGCCCG GCGGCAGCCT | 106 |
| LCA60 | F2 | Heavy | GGTGAAGCCCGGCGGCAGCCTGAGGCTGAGC TGCGAGGCCAGCGGCCTGACCTTCAGCAACGT GTGGATGAGCTGGGTGAGGCAGGCCCCCGGC AAGGGCCTGGAGTGGGTGGGCAGGAT | 107 |
| LCA60 | R1 | Heavy | TGCAGGTACACGGTGTTCTTGCTGTCGTCCCTG CTCAGGGTGAACCTGCCCTTCACGGGGGCGCC GTAGTCGGTGGTGGCGCCCTCGCTCTTCCTCTT GATCCTGCCCACCCACTCCAGG | 108 |
| LCA60 | R2 | Heavy | CCAGTAGTCGAAGTAGTAGCTGCTGCTCCACAC GTCGCCGCCCCTGGTCAGGGTGCTGCAGTAGT ACACGGCGGTGTCGTCGATCTTCAGGCTGTTCA TCTGCAGGTACACGGTGTTCTT | 109 |
| LCA60 | R3 | Heavy | CTAGGTCCCTTGGTCGACGCGCTGCTCACGGT CACCAGGGCGCCCTGGCCCCAGTAGTCGAAGT AGTAG | 110 |
| LCA60 | F1 | Light | CTGCAACCGGTGTACATTCACAGAGCGCCCTG ACCCAGCCCGCCAGCGTGAGCGGCAGCCCCG GCCAGAGCATCACCATCAGCTGCACCGGCACC | 111 |
| LCA60 | F2 | Light | ACCATCAGCTGCACCGGCACCAGCAGCGACGT GGGCACCTACGACCTGGTGAGCTGGTACCAGC AGCACCCCGGCAAGAGCCCCAAGCTGATGATC TACGCCGACATCAAGAGGCCCAGC | 112 |
| LCA60 | R1 | Light | CTGGGGGCGGCCACCGTACGGGTCACCTTGGT GCCGCCGCCGAAGATCACGCTGGTGCTGCTGC CGGCGTACAGGCAGCAGTAGTAGTCGGCC | 113 |
| LCA60 | R2 | Light | GGCAGCAGTAGTAGTCGGCCTCGTCGGCGCTC TGCAGGCCGCTGATGGTCAGGCTGGCGGTGTT GCCGCTCTTGCTGCCGCTGAACCTGTGGCTCA CGCCGCTGGGCCTCTTGATGTCGG | 114 |
| 4C2h | F1 | Heavy | CTGCAACCGGTGTACATTCTCAGGTGCAGCTGC AGGAGAGCGGCGGCGGCCTGGTGAAGCCCGG CGGCAGCCTGAAGCTGAGCTGCGCCGCCAGC GGCTTCACCT | 115 |
| 4C2h | F2 | Heavy | GCGCCGCCAGCGGCTTCACCTTCAGCAGCTAC ACCATGAGCTGGGTGAGGCAGACCCCCGACAA GAGGCTGGAGTGGGTGGCCACCATCAGCAGCG GCGGCAGCTACACCTACTACCCCG | 116 |
| 4C2h | R1 | Heavy | CTAGGTCCCTTGGTCGACGCGCTGCTCACGGT CACCATGGTGCCCTGGCCCCAGTAGTCGTAGT CGTTGCCGTCCCTGGCGCAGTAGTACATGGCG GTGTCCTC | 117 |
| 4C2h | R2 | Heavy | TAGTACATGGCGGTGTCCTCGCTCTTCAGGCTG CTCATCTGCAGGTACAGGGTGTTCTTGGCGTTG TCCCTGCTGATGGTGAACCTGCCCTTCACGCTG TCGGGGTAGTAGGTGTAGCTG | 118 |
| 4C2h | F1 | Light | CTGCAACCGGTGTACATTCAGACATCCAGCTGA CCCAGAGCCCCAGCAGCCTGAGCGCCAGCATC GGCGACAGGGTGACCATCACCTGCCAG | 119 |
| 4C2h | F2 | Light | AGGGTGACCATCACCTGCCAGGCCAGCCAGGA CATCAGCAACTACCTGAACTGGTACCAGCAGAG GCCCGGCCAGGCCCCCAAGCTGCTGATCTACT ACACCAGCAGGCTGCACAGCGGC | 120 |
| 4C2h | R1 | Light | CTGGGGGCGGCCACCGTACGCTTGATCTCCAC CCTGGTGCCGCCGCCGAAGGTCCTGGGCAGG GTGTTGCCCTGCTGGCAGAAGTAGGTG | 121 |
| 4C2h | R2 | Light | CCTGCTGGCAGAAGTAGGTGCCGATGTCCTCG GCCTGGAAGCTGTTGATGGCGATGGTGAAGCT GGTGCCGCTGCCCCTGCCGCTGAACCTGCTGG GCACGCCGCTGTGCAGCCTGCTGG | 122 |
| D12 | F1 | Heavy | CTGCAACCGGTGTACATTCTGAGGTGAAGCTG GTGGAGAGCGGCGGCGGCCTGGTGAAGCCCG GCGGCAGCCTGAAGCTGAGCTGCGCCGCCAG CGGCTTCACCT | 123 |
| D12 | F2 | Heavy | GCGCCGCCAGCGGCTTCACCTTCAGCAGCTAC GCCATGAGCTGGGTGAGGCAGACCCCCGAGAA GAGGCTGGAGTGGGTGGCCACCATCAGCAGCG GCGGCACCTACACCTACTACCCCG | 124 |
| D12 | R1 | Heavy | CTAGGTCCCTTGGTCGACGCGCTGCTCACGGT CACGCTGGTGCCCTGGCCCCAGTAGTCCATGC TGTTGCCGTCCCTCACGCAGTAGTACATGGCG GTGTCCTC | 125 |
| D12 | R2 | Heavy | TAGTACATGGCGGTGTCCTCGCTCCTCAGGCT GCTCATCTGCAGGTACAGGGTGTTCTCGGCGTT GTCCCTGCTGATGGTGAACCTGCCCTTCACGCT GTCGGGGTAGTAGGTGTAGGTG | 126 |
| D12 | F1 | Light | CTGCAACCGGTGTACATTCAGACATCCAGATGA CCCAGACCACCAGCAGCCTGAGCGCCAGCCTG GGCGACAGGGTGACCATCATCTGCAGG | 127 |
| D12 | F2 | Light | AGGGTGACCATCATCTGCAGGGCCAGCCAGGA CATCAACAACTACCTGAACTGGTACCAGAAGCC CGACGGCACCGTGAAGCTGCTGATCTACTACA CCAGCAGGCTGCACAGCGGC | 128 |
| D12 | R1 | Light | CTGGGGGCGGCCACCGTACGCCTCAGCTCCAG CTTGGTGCCGGCGCCGAAGGTGGGGGGCAGG GTGTTGGCCTGCTGGCAGAAGTAGGTG | 129 |
| D12 | R2 | Light | CCTGCTGGCAGAAGTAGGTGGCGATGTCCTCC TGCTCCAGGTTGCTGATGGTCAGGCTGTAGTC GCTGCCGCTGCCGCTGCCGCTGAACCTGCTGG GCACGCCGCTGTGCAGCCTGCTGG | 130 |
| MERS-4V2 | F1 | Heavy | CTGCAACCGGTGTACATTCTGAGGTGCAGCTG GTGGAGAGCGGCGGCGGCCTGGTGCAGCCCG GCAGGAGCCTGAGGCTGAGCTGCGCCGCCAG CGGCT | 131 |
| MERS-4V2 | F2 | Heavy | TGAGCTGCGCCGCCAGCGGCTTCACCTTCAGC AACTACGCCATGTACTGGGTGAGGCAGGCCCC CGGCAAGGGCCTGGAGTGGGTGGCCCTGATCA GCTACGACATCAGCACCGACTACT | 132 |
| MERS-4V2 | R1 | Heavy | CTAGGTCCCTTGGTCGACGCGCTGCTCACGGT CACCAGGGTGCCCTGGCCCCAGTAGTAGGTGT TGGTGCAGTAGTACAGGGCGGTGTCCTCGGTC CT | 133 |
| MERS-4V2 | R2 | Heavy | AGGGCGGTGTCCTCGGTCCTCAGGTTGTTCAT CTGCAGGTAGATGGTGTTCTTGCTGTTGTCCCT GCTGATGGTGAACCTGCCCTTCACGCTGTCGG CGTAGTAGTCGGTGCTGATGTCG | 134 |
| MERS-4V2 | F1 | Light | CTGCAACCGGTGTACATTCACAGAGCGTGCTGA CCCAGAGCCCCAGCGCCAGCGGCACCCCCGG CCAGAGGGTGACCATCAGCTGCAGCGGCAGCA | 135 |
| MERS-4V2 | F2 | Light | CCATCAGCTGCAGCGGCAGCAGCAGCAACATC GGCAACAACTACGTGTACTGGTACCAGCAGCT GCCCGGCACCGCCCCCAAGCTGCTGATCTACT GGAACGACCAGAGGCCCAGCGGCG | 136 |
| MERS-4V2 | R1 | Light | CTGGGGGCGGCCACCGTACGCAGCACGGTCA GCTGGGTGCCGCCGCCGAACACGGCGCCGCT CAGGCTGTCGTCCCAGGCGGCGCAGTAGTAGT C | 137 |
| MERS-4V2 | R2 | Light | CAGGCGGCGCAGTAGTAGTCGGCCTCGTCCTC GCTCCTCAGGCCGCTGATGGCCAGGCTGGCGC TGGTGCCGCTCTTGCTGCCGCTGAACCTGTCG GGCACGCCGCTGGGCCTCTGGTCG | 138 |
| **Table 1. Primer sequences for generation of monoclonal antibody constructs by overlapping PCR** | | | | |

**Table 2:**

| **Monoclonal antibody** | **Heavy chain insert** | **SEQ ID NO:** | **Light chain insert** | **SEQ ID NO:** |
|---|---|---|---|---|
| LCA60 | EVQLLESGGGLVKPGGSLRLSC EASGLTFSNVWMSWVRQAPGK GLEWVGRIKRKSEGATTDYGAP VKGRFTLSRDDSKNTVYLQMNS LKIDDTAVYYCSTLTRGGDVWS SSYYFDYWGQGALVTVSS | 139 | QSALTQPASVSGSPGQSITI SCTGTSSDVGTYDLVSWY QQHPGKSPKLMIYADIKRP SGVSHRFSGSKSGNTASLT ISGLQSADEADYYCCLYAG SSTSVIFGGGTKVT | 140 |
| 4C2h | QVQLQESGGGLVKPGGSLKL SCAASGFTFSSYTMSWVRQT PDKRLEWVATISSGGSYTYYP DSVKGRFTISRDNAKNTLYLQ MSSLKSEDTAMYYCARDGND Y DYWGQGTMVTVSS | 141 | DIQLTQSPSSLSASIGDRV TITCQASQDISNYLNWYQ QRPGQAPKLLIYYTSRLH SGVPSRFSGRGSGTSFTI AINSFQAEDIGTYFCQQG NTLPRTFGGGTRVEIK | 142 |
| D12 | EVKLVESGGGLVKPGGSLKLS CAASGFTFSSYAMSWVRQTP EKRLEWVATISSGGTYTYYPD SVKGRFTISRDNAENTLYLQM SSLRSEDTAMYYCVRDGNSM DYWGQGTSVTVSS | 143 | DIQMTQTTSSLSASLGDRV TIICRASQDINNYLNWYOQK PDGTVKLLIYYTSRLHSGVP SRFSGSGSGSDYSLTISNL EQEDIATYFCQQANTLPPTF GAGTKLELR | 144 |
| MERS-4V2 | EVQLVESGGGLVQPGRSLRLS CAASGFTFSNYAMYWVRQAP GKGLEWVALISYDISTDYYADS | 145 | QSVLTQSPSASGTPGQRVT ISCSGSSSNIGNNYVYWYQ QLPGTAPKLLIYWNDQRPS GVPDRFSGSKSGTSASLAI | 146 |
| | VKGRFTISRDNSKNTIYLQMN NLRTEDTALYYCTNTYYWGQ GTLVTVSS | | SGLRSEDEADYYCAAWDD SLSGAVFGGGTQLTVL | |
| **Table 2. Synthetic V(D)J inserts generated by overlapping PCR.** LCA60 sequences were obtained from PDB file 6nb3 (doi: 10.1016/j.cell.2018.12.028); 4C2h sequences were obtained from Li et al; 2015 (doi: 0.1038/cr.2015.113); D12 sequences were obtained from PDB file 4zpt (doi: 10.1038/ncomms8712), MERS-4V2 sequences were obtained from Zhang et al.; 2018 (doi: 10.1016/j.celrep.2018.06.041). | | | | |

### 2. DPP4 assay:

### a. Pipetting protocol

Enzymatic activity of soluble DPP4 (sDPP4) in serum was measured as follows:
First, in a 96-well plate (Sigma, #M0812-100EA) 10 µL of serum samples were supplied with 90 µl assay buffer (0.5 mM Gly-Pro p-nitroanilide (Biozol, #BAC-4025614.0250) in 50 mM TrisHCl, pH 9.0). Then, samples were measured by the absorbance at 405 nm in a kinetic measurement at 37 °C for 1 hour measuring every 15 minutes (BioTek, Cytation 5). Absorbance was compared to defined DPP4 concentrations by including recombinant DPP4 standards on each plate; prepared as indicated in table 4. Recombinant DPP4 was both produced in the lab and purchased (Sigma-Aldrich Chemie, #D3446). Lab-made DPP4 and commercially available DPP4 showed the same activity.

**Table 3**

| **Standard conc. in end volume (ng/ml)** | **Vol. of 24 ng/µl standard stock solution (µl)** | **1%PBS/BSA (µl)** |
|---|---|---|
| 1400 | 8.60 | 141.40 |
| 1240 | 7.60 | 142.40 |
| 1080 | 6.63 | 143.37 |
| 920 | 5.65 | 144.35 |
| 760 | 4.67 | 145.33 |
| 600 | 3.68 | 146.32 |
| 440 | 2.70 | 147.30 |
| 280 | 1.72 | 148.28 |
| **Table 3. Preparation of 200 µl of recombinant DPP4 standards** | | |

### b. Analysis

The analysis of DPP4 activity assay was performed with RStudio.

Firstly, the mean of two blank values were subtracted from all of the values for each time measurement.

Intercept-free linear regression of known concentrations of recombinant DPP4 versus the absorbance at 405 nm was performed at determined time points. The slope obtained was then used to measure the concentration of DPP4 in the samples.

High concentrations of DPP4 standards show a linear increase of absorbance between 15-60 minutes. Therefore, optimal substrate cleavage for high as well as low concentrations of DPP4 ranges between 45-60 minutes. Later timepoints are not optimal for high concentrations of DPP4. Optimal substrate cleavage is defined by r-squared of the linear regression of the concentrations of DPP4 versus activity higher than 0.96. Samples containing low amount of DPP4 should be measured at later time points; High concentrated control DPP4 samples are eventually excluded from the analysis.

Intercept-free linear regression of known concentrations of DPP4 versus pNA released at optimal time points were performed. The slope was used to measure the concentration of active DPP4 (aDPP4) in the samples.

### 3. ELISA:

**Table 4:**

| **Protein** | **Information** | **Size (RBD/S1S2)** | **Extinction coefficient (RBD/S1S2)** |
|---|---|---|---|
| MERS-CoV_WT | Twin-Strep, His | RBD: ∼ 32 kDa | RBD: 12250 |
| | | S1S2: ~ 148 kDa | S1S2:77000 |
| | His | RBD: ~ 27 kDa | RBD: 34630 |
| | | S1S2: ~ 145 kDa | S1S2: 155323 |
| MERS-CoV_V555Y | Twin-Strep, His | RBD: ~ 32 kDa | RBD: 51130 |
| | | S1S2: ~ 148 kDa | S1S2: 166323 |
| | His | RBD: ~ 27 kDa | RBD: 36120 |
| | | S1S2: ~ 145 kDa | S1S2: 156813 |
| MERS-CoV _L506R | Twin-Strep, His | RBD: ~ 32 kDa | RBD: 51130 |
| | | S1S2: ~ 148 kDa | S1S2: 166323 |
| | His | RBD: ~ 27 kDa | RBD: 36120 |
| | | S1S2: ~ 145 kDa | S1S2: 156813 |
| MERS-CoV_V555W | Twin-Strep, His | RBD: ~ 32 kDa | RBD: 51130 |
| | | S1S2: ~ 148 kDa | S1S2: 166323 |
| MERS-CoV_V555R | Twin-Strep, His | RBD: ~ 32 kDa | RBD: 51130 |
| | | S1S2: ~ 148 kDa | S1S2: 166323 |
| MERS-CoV_V555F | Twin-Strep, His | RBD: ∼ 32 kDa | RBD: 51130 |
| | | S1S2: ∼ 148 kDa | S1S2: 166323 |
| MERS-CoV_V555A | Twin-Strep, His | RBD: ∼ 32 kDa | RBD: 51130 |
| | | S1S2: ∼ 148 kDa | S1S2: 166323 |
| MERS-CoV_G538E | Twin-Strep, His | RBD: ∼ 32 kDa | RBD: 51130 |
| | | S1S2: ∼ 148 kDa | S1S2: 166323 |
| MERS-CoV_G538K | Twin-Strep, His | RBD: ∼ 32 kDa | RBD: 51130 |
| | | S1S2: ∼ 148 kDa | S1S2: 166323 |
| MERS-CoV_G538H | Twin-Strep, His | RBD: ∼ 32 kDa | RBD: 51130 |
| | | S1S2: ∼ 148 kDa | S1S2: 166323 |
| MERS-CoV_G538W | Twin-Strep, His | RBD: ∼ 32 kDa | RBD: 51130 |
| | | S1S2: ∼ 148 kDa | S1S2: 166323 |
| MERS-CoV_G462L | Twin-Strep, His | RBD: ∼ 32 kDa | RBD: 51130 |
| | | S1S2: ∼ 148 kDa | S1S2: 166323 |
| MERS-CoV_G462Q | Twin-Strep, His | RBD: ∼ 32 kDa | RBD: 51130 |
| | | S1S2: ∼ 148 kDa | S1S2: 166323 |
| MERS-CoV_G462R | Twin-Strep, His | RBD: ∼ 32 kDa | RBD: 51130 |
| | | S1S2: ∼ 148 kDa | S1S2: 166323 |
| MERS-CoV-S1S2_VPP | Twin-Strep, His | ~ 148 kDa | 166385 |
| MERS-CoV-S1S2_TS | Twin-Strep, His | ~ 148 kDa | 166385 |
| MERS-CoV-S1S2_R307A_V555Y | His | ~ 145 kDa | 156813 |
| MERS-CoV-S1S2_F39A* | Twin-Strep, His | ~ 148 kDa | 166385 |
| MERS-CoV-S1S2_S133A* | Twin-Strep, His | ~ 148 kDa | 166385 |
| MERS-CoV-S1S2_R307A* | Twin-Strep, His | ~ 148 kDa | 166385 |
| MERS-CoV-S1S2_F39A_V555Y* | Twin-Strep, His | ~ 148 kDa | 166385 |
| MERS-CoV-S1S2_S133A_V555Y* | Twin-Strep, His | ~ 148kDa | 166385 |
| MERS-CoV_S460W* | Twin-Strep, His | RBD: ~ 32 kDa | RBD: 51130 |
| | | S1S2: ~ 148 kDa | S1S2: 166323 |
| MERS-CoV_G462W* | Twin-Strep, His | RBD: ~ 32 kDa | RBD: 51130 |
| | | S1S2: ~ 148 kDa | S1S2: 166323 |
| MERS-CoV _G462Y* | Twin-Strep, His | RBD: ~ 32 kDa | RBD: 51130 |
| | | S1S2: ~ 148 kDa | S1S2: 166323 |
| MERS-CoV _G462F* | Twin-Strep, His | RBD: ~ 32 kDa | RBD: 51130 |
| | | S1S2: ~ 148 kDa | S1S2: 166323 |
| MERS-CoV _S498W* | Twin-Strep, His | RBD: ~ 32 kDa | RBD: 51130 |
| | | S1S2: ~ 148 kDa | S1S2: 166323 |
| MERS-CoV _S504W* | Twin-Strep, His | RBD: ~ 32 kDa | RBD: 51130 |
| | | S1S2: ~ 148 kDa | S1S2: 166323 |
| MERS-CoV _R505L* | Twin-Strep, His | RBD: ~ 32 kDa | RBD: 51130 |
| | | S1S2: ~ 148 kDa | S1S2: 166323 |
| MERS-CoV _L506W* | Twin-Strep, His | RBD: ~ 32 kDa | RBD: 51130 |
| | | S1S2: ~ 148 kDa | S1S2: 166323 |
| MERS-CoV _L506Y* | Twin-Strep, His | RBD: ~ 32 kDa | RBD: 51130 |
| | | S1S2: ~ 148 kDa | S1S2: 166323 |
| MERS-CoV _L506H* | Twin-Strep, His | RBD: ~ 32 kDa | RBD: 51130 |
| | | S1S2: ~ 148 kDa | S1S2: 166323 |
| MERS-CoV _L534W* | Twin-Strep, His | RBD: ~ 32 kDa | RBD: 51130 |
| | | S1S2: ~ 148 kDa | S1S2: 166323 |
| MERS-CoV _G538V* | Twin-Strep, His | RBD: ~ 32 kDa | RBD: 51130 |
| | | S1S2: ~ 148 kDa | S1S2: 166323 |
| MERS-CoV _G538A* | Twin-Strep, His | RBD: ~ 32 kDa | RBD: 51130 |
| | | S1S2: ~ 148 kDa | S1S2: 166323 |
| MERS-CoV _G538D* | Twin-Strep, His | RBD: ~ 32 kDa | RBD: 51130 |
| | | S1S2: ~ 148 kDa | S1S2: 166323 |
| MERS-CoV _G538F* | Twin-Strep, His | RBD: ~ 32 kDa | RBD: 51130 |
| | | S1S2: ~ 148 kDa | S1S2: 166323 |
| MERS-CoV _G538L* | Twin-Strep, His | RBD: ~ 32 kDa | RBD: 51130 |
| | | S1S2: ~ 148 kDa | S1S2: 166323 |
| MERS-CoV _G538S* | Twin-Strep, His | RBD: ~ 32 kDa | RBD: 51130 |
| | | S1S2: ~ 148 kDa | S1S2: 166323 |
| MERS-CoV _G538Y* | Twin-Strep, His | RBD: ~ 32 kDa | RBD: 51130 |
| | | S1S2: ~ 148 kDa | S1S2: 166323 |
| MERS-CoV _G538C* | Twin-Strep, His | RBD: ~ 32 kDa | RBD: 51130 |
| | | S1S2: ~ 148 kDa | S1S2: 166323 |
| MERS-CoV _G538P* | Twin-Strep, His | RBD: ~ 32 kDa | RBD: 51130 |
| | | S1S2: ~ 148 kDa | S1S2: 166323 |
| MERS-CoV _G538Q* | Twin-Strep, His | RBD: ~ 32 kDa | RBD: 51130 |
| | | S1S2: ~ 148 kDa | S1S2: 166323 |
| MERS-CoV _G538I* | Twin-Strep, His | RBD: ~ 32 kDa | RBD: 51130 |
| | | S1S2: ~ 148 kDa | S1S2: 166323 |
| MERS-CoV _G538M* | Twin-Strep, His | RBD: ~ 32 kDa | RBD: 51130 |
| | | S1S2: ~ 148 kDa | S1S2: 166323 |
| MERS-CoV_G538T* | Twin-Strep, His | RBD: ~ 32 kDa | RBD: 51130 |
| | | S1S2: ~ 148 kDa | S1S2: 166323 |
| MERS-CoV _G538N* | Twin-Strep, His | RBD: ~ 32 kDa | RBD: 51130 |
| | | S1S2: ~ 148 kDa | S1S2: 166323 |
| MERS-CoV _V555K* | Twin-Strep, His | RBD: ~ 32 kDa | RBD: 51130 |
| | | S1S2: ~ 148 kDa | S1S2: 166323 |
| MERS-CoV _A556W* | Twin-Strep, His | RBD: ~ 32 kDa | RBD: 51130 |
| | | S1S2: ∼ 148 kDa | S1S2: 166323 |
| MERS-CoV _S557Y* | Twin-Strep, His | RBD: ∼ 32 kDa | RBD: 51130 |
| | | S1S2: ∼ 148 kDa | S1S2: 166323 |
| MERS-CoV _S557W* | Twin-Strep, His | RBD: ∼ 32 kDa | RBD: 51130 |
| | | S1S2: ∼ 148 kDa | S1S2: 166323 |
| MERS-CoV _S557F* | Twin-Strep, His | RBD: ∼ 32 kDa | RBD: 51130 |
| | | S1S2: ∼ 148 kDa | S1S2: 166323 |
| MERS-CoV _S559W* | Twin-Strep, His | RBD: ∼ 32 kDa | RBD: 51130 |
| | | S1S2: ∼ 148 kDa | S1S2: 166323 |
| BG505 | Twin-Strep, His | ∼ 140 kDa | 147397 |
| Human DPP4 | His | ∼ 86 kDa | 193815 |
| Rabbit DPP4 | His | ∼ 8 kDa | 193730 |
| SARS2-CoV | Twin-Strep, His | RBD: ∼ 29 kDa | RBD: 44550 |
| | | S1S2: ∼ 180 kDa | S1S2: 70250 |
| LCA60 | IgG | ~ 150 kDa | ~210000 |
| D12 | IgG | ~ 150 kDa | ~210000 |
| MERS4V | IgG | ~ 150 kDa | ~210000 |
| h4C2 | IgG | ~ 150 kDa | ~210000 |
| G4 | IgG | ~ 150 kDa | ~210000 |
| **Table 4. Protein information. Extinction coefficient estimated by ProtParam tool of Expasy.** *Data from proteins still to be included in the patent. | | | |

### a. IgG- concentration ELISA:

Concentration of self-made antibodies was determined using ELISA. Goat anti-IgG human (Southern Biotech, #2040-04) was used to coat a plate using 25 µl of 2 µg/ml in PBS. The plate was incubated at 4 °C overnight.

The plate was washed three times with PBS-T and blocked using 50 µl PBS 1% BSA for 1 hour at RT. The plate was washed three times with PBS-T and 25 µl of serial dilutions of the samples (starting dilution 1 :200) and a positive control (Southern Biotech, #0150-01, starting concentration 2 µg/ml) were added and incubated for 1 hour at RT. Then, the plate was washed three times with PBS-T and 25 µl of goat anti-human IgG (Southern Biotech, #2040-04) was added to each well and incubated for 1 hour at RT. Plates were washed three times with PBS-T and 50 µl pNPP (Sigma, #S0492-50TAB) was added. Exactly after 30 minutes of pNPP addition, absorbance of wells at 405 nm and 620 nm was measured (BioTek, Cytation 5).

### b. DPP4 concentration ELISA:

To analyse binding of MERS-CoV-RBD to MERS-CoV-spike to DPP4 by ELISA, 96 well half-area plates (Greiner Bio-One GmbH, #675061) were coated using 25 µl of 4 µg/ml self-made DPP4 per well followed by incubation overnight at 4 °C.

Then plates were washed three times with PBS-T (Biotek, EL406). Plates were blocked with 25 µl of PBS 1% BSA for one hour at RT. The plates were washed three times with PBS-T and 25 µl of serial dilutions of MERS-CoV-RBD or MERS-CoV-spike proteins were added to each well and incubated for 1 hour at RT. Serial dilutions were generated with a three-fold dilution with distinct starting concentration of recombinant protein. Plates were washed three times with PBS-T. 25 µl of 2 µg/ml AP-coupled Streptavidin (Southern Biotech, #0150-01) were added to each well and incubated for 1 hour at RT. Plates were washed three times with PBS-T and 50 µL pNPP (Sigma, #S0492-50TAB) was added. Exactly after 30 minutes of pNPP addition, absorbance of wells at 405 nm and 620 nm was measured (BioTek, Cytation 5). Increased absorbance at 405 nm indicated DPP4 binding to the sample Twin Strep tagged protein.

### c. Affinity ELISA:

Binding of MERS-CoV variants to DPP4 and neutralizing antibodies (nAbs) was assessed using affinity ELISA. In both cases, 96 well half-area plates (Greiner Bio-One GmbH, #675061) were coated using 25 µl of 4 µg/ml desired MERS-CoV variant and incubated overnight at 4 °C.

Then plates were washed three times with PBS-T (Biotek, EL406). Plates were blocked with 25 µl of PBS 1% BSA for one hour at RT. The plates were washed three times with PBS-T. **DPP4 binding** was measured adding 25 µl of serial dilutions of rabbit or human DPP4 to each well and incubated for 1 hour at RT. Serial dilutions were generated with three-fold serial dilutions of distinct starting concentrations of human recombiant DPP4 (eg. 3 µg/ml) and rabbit DPP4 (eg. 60 µg/ml). Plates were washed three times with PBS-T and 25 µl of 0.6 µg/ml goat polyclonal anti-DPP4 (Biotechne, #AF1180) was added to each well and incubated for 1 hour at RT. Plates were washed three times with PBS-T. 25 µl of 2 µg/ml AP-coupled donkey anti-goat IgG (Dianova, #705-056-147) were added to each well and incubated for 1 hour at RT. Plates were washed three times with PBS-T and 50 µL pNPP (Sigma, #S0492-50TAB) were added. Exactly after 30 minutes of pNPP addition, absorbance of wells at 405 nm and 620 nm was measured (BioTek, Cytation 5). **nAb binding** was measured adding 25 µl of three-fold serial dilution of 1 µg/ml starting concentrations of different nAbs (Table 3) to each well followed by incubation for 1 hour at RT. Plates were washed three times with PBS-T and 25 µl of 2 µg/ml AP-coupled goat anti-human IgG (Jackson ImmunoResearch, #109-056-098) were added to each well and incubated for 1 hour at RT. Plates were washed three times with PBS-T and 50 µL pNPP (Sigma, #S0492-50TAB) were added. Exactly after 30 minutes of pNPP addition, absorbance of wells at 405 nm and 620 nm is measured (BioTek, Cytation 5).

### d. Rabbit IgG ELISA:

Quantification of rabbit IgG antibodies reactive against MERS-CoV RBD and MERS-CoV SPIKEsubunits was assessed by ELISA. 96 well half-area plates (Greiner Bio-One GmbH, #675061) were coated using 25 µl of 4 µg/ml desired MERS-CoV RBD His or MERS-CoV spike His and incubated overnight at 4 °C.

Then plates were washed three times with PBS-T (Biotek, EL406). Plates were blocked with 25 µl of PBS 1% BSA for one hour at RT. The plates were washed three times with PBS-T and 25 µl of three-fold serial dilutions (starting at 1 :500) of rabbit serum were added to each well and incubated for 1 hour at RT. Plates were washed three times with PBS-T and 25 µl of 2 µg/ml AP-coupled goat anti-rabbit (Jackson ImmunoResearch, #111-056-003) were added to each well and incubated for 1 hour at RT. Plates were washed three times with PBS-T and 50 µL pNPP (Sigma, #S0492-50TAB) were added. Exactly after 30 minutes of pNPP addition, absorbance of wells at 405 nm and 620 nm was measured (BioTek, Cytation 5).

Analysis of rabbit anti-MERS-CoV RBD/spike IgG titers was measured using area under curve (AUC) or effective dilution 50 (ED50), the dilution at which 50% of maximum IgG is bound to the antigen. Calculations were done using RStudio and Prism^{®}.

### e. DPP4 Competition ELISA:

The potential of antibodies to block DPP4 binding the MERS-CoV RBD was assessed by competition ELISA.

DPP4 was biotinylated using the kit EZ-Link^{™} NHS-PEG Solid-Phase Biotinylation Kit (ThermoFisher, #21450).

96 well half-area plates (Greiner Bio-One GmbH, #675061) were coated using 25 µl of 4 µg/ml MERS-CoV spike His and incubated overnight at 4 °C. Then plates were washed three times with PBS-T (Biotek, EL406). Plates were blocked with 25 µl of PBS 1% BSA for one hour at RT. The plates were washed three times with PBS-T and 25 µl of serial dilutions of rabbit serum were added to each well and incubated for 1 hour at RT. Serial dilutions were generated with a dilution factor of 1.7 and a starting dilution of 1 to 10. Then addition of 25 µl of 2XEC70 biotinylated-DPP4 were added and incubated for 1 hour at RT. Plates were washed three times with PBS-T and 25 µl of AP-coupled Streptavidin (Southern Biotech, #0150-01) were added to each well and incubated for 1 hour at RT. Plates were washed three times with PBS-T and 50 µL pNPP (Sigma, #S0492-50TAB) were added. Exactly after 30 minutes of pNPP addition, absorbance of wells at 405 nm and 620 nm was measured (BioTek, Cytation 5).

### 4. Protein production and purification

HEK293 cells were cultured in at 37°C in a humidified 8% CO2 incubator. For protein production, cells were grown to a density of 2*10⁶ cells per ml, transfected using 1 mg/ml PEI (Polysciences Inc., # 23966-1) and 38 µg plasmid DNA, and cultivated for 3 days. Supernatant was collected and filtrated using 0.45 µm Polyethersulfone membranes. Proteins were purified from the supernatants by His SpinTrapTM columns according to manufacturer's instructions (Cytiva, #95056-290). Eluted proteins were transferred to phosphate-buffered saline (PBS) via buffer exchange. Buffer exchange comprises six consecutive concentration and dilution steps at 4 °C in a centrifuge for 4-14 min at 4000 g using Amicon Ultra-4 centrifugal filter units with 10 kDa cutoff or 50 kDa (Millipore; #UFC801008, #UFC805008), for RBD proteins or hDPP4-N'His and monoclonal antibodies, respectively. Protein concentration was determined by ELISA for all proteins. Protein production was confirmed by SDS-PAGE and Western Blot.

### 5. Serum DPP4 pull-down

Soluble DPP4 was pull down from 200 µl of serum using MERS-RBD-TwinStrep coupled to streptavidin magnetic beads (IBA Life Science, #2-4090-010). First, 120 µl of streptavidin magnetic beads was placed in a 1,5 ml tube. Using a magnetic rack, streptavidin magnetic beads were separated from the liquid. Liquid was removed and the pellets beads were washed twice with 1 ml 1 xWash buffer (IBA Life Science, #2-1003-100) and twice with 1 ml PBS. After washing, beads were resuspended in 50 µl of PBS.

TwinStrep containing proteins were coupled to streptavidin magnetic beads by addition of 24 µg of desired protein and incubation of the solution at 37°C for 1 hour shaking at 1400 rpm (Starlab, #S8012-0000). After 1 hour, beads were washed three times with 1 ml PBS using the magnetic rack to pellet and exchange the supernatant. Blocking of the beads was necessary to occupy potentially free spaces on the streptavidin magnetic beads. Beads wre blocked with 1 ml 1% BSA-PBS for 1 hour at RT shaking at 1400 rpm.

After the blocking step, beads were washed three times with 1 ml PBS and finally resuspended in 120 µl of PBS. 120 µl of beads was transferred to a 2 ml tube. 200 µl of serum was added to the 2 ml tube. The 2 ml tube was placed inside a 50 ml tube together with paper towels for fixation. The 50 ml tube was incubated overnight at 4°C rolling.

Following the overnight incubation, 2 ml tubes were placed in the magnetic rack and serum was collected for further analysis. Pellet beads were washed three times with 1 ml of PBS and stored for further analysis.

### 6. Western blot

Preparation of samples for SDS-PAGE started with mixing the desired amount of proteins with 1 x Laemmli buffer (Biozol Diagnostica, #GTX16357-25) containing 5% 2-mercaptoethanol (Thermo Fisher, #31350010). Samples were then denatured for 5 min at 95 °C in a thermal cycler. Samples containing magnetic beads were denatured for 30 min at 95 °C to ensure optimal protein extraction.

SDS-PAGE was performed using 10 wells- 4-15 % TGX Stain free gels (#4568083, Bio-Rad) in a the Mini-PROTEAN Tetra Cell (Bio-Rad, #1658004) built with gel running modules (Bio-Rad, #1658038), with 1X TGS buffer (Bio-Rad, #1610732) as running buffer.

Denatured samples were loaded in the wells (up to 25 µl). Beads were removed from samples before loading. Protein standards (Bio-Rad, #1610374) were used to mark protein sizes. Once samples were loaded on the gel, the chamber was closed and plugged to a power supply (Bio-Rad, #1645050). Gels were run at 80 V for the first 15 min. Later voltage was raised to 300 V for 30 min until completion of the run.

Proteins were transferred from SDS-Gel to a nitrocellulose membrane. The sponge and the membrane from the nitrocellulose transfer pack (Bio-Rad, #1704159) were placed in the middle of a cassette from the Trans-Blot Turbo System (Bio-Rad, #1704150). Gels were extracted from the pre-cast plastic and placed on top of the nitrocellulose membrane in one move. The other sponge from the pack was placed on top of the gel. Using a blot roller (Bio-Rad, #1651279), potential bubbles between the gel and the nitrocellulose membrane were removed. The cassette was then closed in one move and inserted in the Trans-Blot Turbo System. Protein transfer was done using the MINI-GEL program, consisting of a transfer for 3 min at constant 2.5 A up to 25 V.

Once the transfer of proteins was completed, the nitrocellulose membrane was incubated in Ponceau solution (Sigma, #P7170) to visualize successful transfer. Nitrocellulose membrane was washed 3 times for 5 min with 30 ml TBST (1% Tween, 150 µM NaCl, 20 µM Tris, pH 7.6). The membrane was blocked with 30 ml of 5% milk (Roth, #T145.2) in TBST for 30 min. Then the membrane was placed in a 50 ml tube containing the desired primary antibody diluted in 3 ml of 5% milk in TBST. The antibodies used in this project were: goat polyclonal anti- human DPP4 (R&D Systems, #AF1180) at 0.2 µg/ml and mouse anti-6xHis tag (Abcam, #ab18184) at 1 µg/ml. Primary antibody incubation was performed overnight at 4 °C rolling. On the next day, the membrane was washed three times with 30 ml TBST for 5 min and incubated with the secondary antibody. The secondary antibody was either IRDye^{®} 800CW donkey anti-goat IgG (Li-Cor Biosciences, #925-32214 or donkey anti-mouse IgG (Li-Cor Bioscience, #925-32212), for goat anti-DPP4 antibody and mouse anti-His antibody; respectively. The membrane was incubated for 2 hours in a 50 ml tube at RT in 5 ml of TBST containing the secondary antibody at 0.2 µg/ml rolling.

After the secondary antibody incubation was finished, the membrane was washed three times with 30 ml of TBST for 5 minutes. The membrane was scanned in an infrared membrane scanner (Li-Cor Odyssey IR scanner).

### 7. Pseudovirus neutralization assay:

For MERS-CoV spike pseudotyped viral particle production. HEK293T cells were cultivated until confluent in T175 flasks (SARSTEDT, #83.3912.002), washed with 10 ml PBS-T (Biotek, #EL406) and detached using 3 ml of Trypsin EDTA (Sigma Aldrich, #T4049). Trypsinisation was blocked using 12 ml of DMEM complete (10 % FBS, 1% Pen-Strep DMEM (Life Technology, #10566016)), cells were resuspended and counted. 50 million cells were supplied with 160 ml of DMEM and the cell-solution was distributed to 20 tissue culture dishes (SARSTEDT, #83.3902) (2.5 million cells per 8 ml per dish).

Next day the transfection reagent was prepared by mixing 1.7 ml of CaCl2 solution with 12.3 ml of water in a 50 ml falcon and adding the DNA plasmids accordingly:
- 100 µg *pCSII-Luc (lentiviral vector genome encoding luciferase)*
- 200 µg pCMV delta R8.2 (addgene #12263, lentiviral packaging plasmid, containing HIV-1 GAG/POL, Tat and Rev)
- 10 µg pcDNA3_MERS_S1S2_TM_d19 (plasmid expressing MERS-CoV spike protein with transmembrane domain and cytoplasmic tail, 19AA truncated)

The plasmid mixture was added dropwise into a 14 ml of 2xHEPES-Buffered saline from the transfection reagent kit (Takara, #631312) while agitating with an electronic pipet using air bubbles and incubated for 20 minutes at room temperature. The HEK293T medium was exchanged and 1.4 ml of transfection mixture was added drop-wise into each dish.

The medium was exchanged 16 hours later. Viral supernatant was harvested 48 and 72 hours after transfection by transferring the medium to falcon tubes. The supernatant was clarified by filtration through 0.45 µm Polyethersulfone membranes (Th.Geyer Berlin GmbH, #9049912).

The MERs-CoV pseudotyped viral particles were concentrated by ultracentrifugation, distributing 25 ml of virus supernatant in six polypropylene centrifuge tubes (Beckman Coulter, #326819) and adding 5 ml of 20% Sucrose in PBS below the virus supernatant. Tubes were centrifuged for 90 minutes at 4°C at 30.000 rpm (Beckman Coulter L60 Ultracentrifuge) using a swinging-bucket rotor (Beckman Coulter, #369694) to pellet the virus. The supernatant was decanted from the tubes and the pellet was resuspended using 600 µl Hanks' Balanced Salt Solution (Gibco, #14025092) and the pellet was resuspended shaking overnight at 4°C.

One day after the ultracentrifugation step the virus supernatant was aliquoted in 300 µl and stored at -80°C.

MERS-CoV pseudotyped viral particle titration was performed with Huh7 cells (CLS Cell Lines Service GmbH, #300156) which have endogenous DPP4 expression. The assay started preparing a 96 well-plate (SARSTEDT, #833925500) by filling the outer wells with 200 µl of PBS-T to minimize evaporation. In the inner wells of the plate, 12.500 Huh7 cells were seeded per well in 50 µl medium.

The next day, in a 96-wells plate, a threefold serial dilution of eight steps of virus supernatant was prepared in RPMI medium in hexaplicates, starting with a 1 to 3 dilution. A negative control was included in hexaplicate, which is RPMI medium only. The dilutions were added to the Huh7 cells using 50 µl The viral dilutions were incubated on the Huh7 cells for 48 hours at 37 °C, 5% CO2.

Next, 50 µl of virus-serum mixture was added to the cultured Huh7 cells and incubated for 48 hours at 37°C, 5% CO2.

Readout of the assay started by removing 70 µl of medium from each well, leaving around 30 µl of medium. Then 30 µl of luciferase reagent (Promega, #E2610) was added in each well and incubated for 2 minutes in the dark at room temperature allowing complete cell lysis. The sample was mixed and 50 µl was transferred to a white 96-well plate (Neolab, #3688). Luminescence was read 5 minutes after addition of luciferase substrate using Cytation 5 (Biotek).

MERS-CoV pseudotyped viral particle titration titers were calculated by determining 50% tissue culture infectious dose (TCID50) based on the relative light units (RLUs) of each viral dilution according to Nie, J., 2020 (https://doi.org/10.1038/s41596-020-0394-5).

MERS-CoV pseudotyped viral particle neutralization was performed with Huh7 cells. The assay started preparing a 96 well-plate by filling the outer wells with 200 µl of PBS-T to minimize evaporation. In the inner wells of the plate, 12.500 Huh7 cells were seeded per well in 50 µl medium.

The next day, rabbit serum samples were inactivated by transferring 10 µl to a 0.5 mL tube and incubating for 1 hour at 56 °C in a water bath. Next, in a 96-wells plate, with the outer wells filled by 200 µl of PBS-T, a five-fold serial dilution of six steps of rabbit serum samples was prepared in duplicates in RPMI medium, starting with a 1 to 10 dilution with an end volume of 25 µl. To each well, 100 TCID50 of MERS-CoV pseudotyped viral particles was added, diluted in RPMI medium with an end volume of 50 µl in each well. A negative control (cell control) was included in hexaplicate, which is RPMI medium only as well as a positive control (virus control) in hexaplicate, which is 100 TCID50 of MERS-CoV pseudotyped viral particles, both with an end volume of µl in each well. The serum dilutions with MERS-CoV pseudovirus were incubated 1 hour at 37 °C, 5% CO2. The dilutions were added to the Huh7 cells using 50 µl Following this, the viral dilutions were incubated on the Huh7 cells for 48 hours at 37 °C, 5% CO2.

Readout of the assay started by removing 70 µl of medium from each well, leaving around 30 µl of medium. Then 30 µl of luciferase reagent (Promega, #E2610) was added in each well and incubated for 2 minutes in the dark at room temperature allowing complete cell lysis. The sample was mixed and 50 µl was transferred to a white 96-well plate (Neolab, #3688). Luminescence was read 5 minutes after addition of luciferase substrate using Cytation 5 (Biotek).

MERS-CoV pseudotyped viral particle neutralization titers were calculated by first normalizing the relative light units (RLUs) for each rabbit serum replicate to the cell control and virus control. Then the neutralization percentage curves were calculated using log(inhibitor) vs. normalized response - variable slope using GraphPad Prism, according to Ferrara and Temperton (F. Ferrara and N. Temperton, "Pseudotype Neutralization Assays: From Laboratory Bench to Data Analysis," Methods and protocols, vol. 1, no. 1, pp. 1-16, 2018).

### 8. Biolayer Interferometry:

Anti-Penta-His biosensors were equilibrated with 10 µg/mL MERS-CoV RBD Twin- Strep His for 300 s prior to association. Association and dissociation were both done for 300 s. The dissociation constant was determined by global fitting 1:1, with step correction for start of association and start of dissociation in BLItz Pro 1.2.0.49 (ForteBio). Visualization was done in GraphPad Prism with normalization to a blank control.

### Example 1

To address the abundance of soluble DPP4 in blood samples, previously established enzymatic activity measurement with a fluorescent substrate was applied (E. M.Varin et al.: "Circulating Levels of Soluble Dipeptidyl Peptidase-4 Are Dissociated from Inflammation and Induced by Enzymatic DPP4 Inhibition" Cell Metabolism, Volume 29, Issue 2, 5 February 2019, Pages 320-334.e5, https://www.sciencedirect.com/science/article/pii/S1550413118306338 (Figure 1, A,B). Substrate cleavage velocity in serum and plasma from 90 healthy individuals revealed a median concentration of 639 ng/ ml active enzyme (Figure 1C).

### Example 2

Single point mutations in the MERS-CoV RBM and MERS-CoV spike, capable of abrogating DPP4 binding, have been identified by a contact based analysis of the crystal structure of human DPP4 bound to the MERS-CoV RBD (PDB 4L72). RBD residues at positions 462, 502, 506, 511, 512, 513, 538, 540, 553, and 555 have been identified as top scoring candidates for the abrogation of DPP4 binding to the RBD (Figure 2). Further candidates of this analysis include S460, S498, S504, R505, L506, V534, A556, S557, and S559.

In addition to mutating single amino acids, a distinct design strategy creating a MERS-CoV spike protein, which is locked in a so-called RBD-down-conformation was followed. The lock down of RBDs onto the spike backbone has two functions: i) it stabilizes the protein in a so-called pre-fusion conformation thereby preventing induction of non-functional antibodies raised against the post-fusion conformation, and ii) it prevents the binding to DPP4 as the RBM is not accessible. No MERS-CoV down-lock has been established yet. Positions V458 V1060 L1061 (VPP, fig. 3A) in the MERS-CoV spike have been identified to introduce disulphide bonds by mutating to V458C V1060K L1061C. This introduction of two cysteines is expected to link the RBD and the spike stem (S2) of different monomers together. In addition, T424 and S459 have been identified which can be mutated to link the RBDs to each other by disulphide bonds introducing T424C S459C exchanges (TS, fig. 3B). Both attempts succeeded in producing disulphide-linked MERS-CoV trimers, with markedly different mobility than non-locked MERS-CoV spike in gel electrophoresis under non-reducing and native conditions (Figure 3 C,D).

### Example 3

The DPP4 binding performance of single point mutations and the spike down-locks have been tested *in vitro* by ELISA for mRBD G462R, G462Q, G462L, G538H, G538W, L506R, G538E, G538K, V555W, V555Y, V555R, V555F and mSpike TS, VPP (Figure 4 A, B). Further, the DPP4 binding performance of single point mutations have been tested *in vitro* by ELISA for mRBD G538A, G538F, G538L, G538I, G538D, G538E, G538K, G538H, G538W (Figure 4 C, D). Compared to the wildtype spike, all mRBDs and mSpikes tested decreased binding to human and rabbit DPP4.

G462R, G462Q, G462L mutations decreased binding to human and rabbit DPP4. A substantial abrogation of host receptor binding was observed for G538H and G538W followed by the two down-lock variants TS and VPP. The highest performance for loss of DPP4 binding was observed for L506R, G538E, G538K, G538A, G538F, G538L, G538I, G538D, V555W, V555Y, V555R, and V555F.

### Example 4

The RBM is a crucial target for neutralizing antibodies (nAbs). Point mutations in the RBM preventing receptor binding may consequently destroy nAb epitopes and decrease the induction of nAbs when the mutant antigen is used for immunizations. For SARS-CoV-2, distinct single point mutations were shown to interfere with nAb binding to different degrees. To approximate the impact of selected MERS-CoV mutants on nAb epitopes, 4 neutralizing monoclonals with distinct RBD binding footprints that are structurally defined were recombinantly produced, namely LCA60, D12, 4C2h, and MERS4V2.

It was then tested if selected DPP4-abrogating spike mutants maintain or destroy binding to the 4 monoclonal antibodies (Figure 5 A - E). Overall, TS and VPP down-lock mutants maintained binding to all 4 nAbs, although binding of nAbs MERS4V and 4C2H was impaired, likely because their binding footprint is hidden in down-lock structures. The V555W/R/F/Y, G538H, G538Q as well as L502R mutants maintained strong binding to all monoclonal antibodies (Figure 5A, B, D, E). G538W showed slightly reduced binding of nAb 4C2 while maintaining binding to the other nAbs. Binding of 4C2h is abrogated for G538Y, G538P, G538M, G538V, G538A, G538I or reduced for G538S, G538C, G538N, G538R, G538F, G538L, G538D, G538E, G538H. D12 is also affected for G538S, G538P, G538M, G538T, G538R, G538V, G538A, G538I. All G538 mutants tested maintained binding to LCA60 and MERS4V2 (Figure 5 D, E).

As controls MERS-CoV RBD mutations, E436R, D537K, and D539K have been used and showed severely impaired binding of 3, 1, and 2 nAbs, respectively (Figure 5C).

Together, the findings show that mutations at positions G462, G538, L506, V555 and mSpike TS, VPP, specifically G462R, G462Q, G462L, G538H, G538W, G538Y, G538P, G538M, G538V, G538A, G538I, G538S, G538C, G538N, G538R, G538F, G538L, G538D, G538E, G538K, L506R V555W, V555Y, V555R, V555F and mSpike TS, VPP are good candidates for a vaccine aiming at abrogating host receptor binding to foster neutralizing antibody responses in vivo.

Moreover, mutations at positions L506, G538, and V555, specifically L506R, G538E/K, V555E/Y/R/F are prioritized candidates for a vaccine aiming at abrogating host receptor binding to foster neutralizing antibody responses in vivo.

### Example 5

To identify a suitable animal model for in vivo immunizations, the sequence homology of mouse, rat, and rabbit DPP4 were studied and compared to camel and human, two species which are susceptible to MERS-CoV infection. In the propeller domain of DPP4 ranging from AA253-400 (according to the human sequence) a total of 17, 31, 36, and 49 amino acid exchanges and 2, 3, 4, and 4 exchanges at crucial MERS-CoV contact residues for rabbit, camel, mouse, and rat, were observed respectively. To determine the reactivity of MERS-CoV wt RBD to species specific DPP4, serum pull downs from animal blood samples were performed and it was observed that circulating rabbit DPP4 but not mouse or rat DPP4 is captured by MERS-CoV RBD coated beads. Likewise, an ELISA assay confirmed that rabbit but not mouse DPP4 binds to the MERS-CoV RBD despite a lower binding strength compared to human DPP4 (Figure 6A).

Serum concentration as well as receptor binding affinity of rabbit DPP4 were measured and compared to its human counterpart. Both factors are crucial determinants for epitope occupancy by soluble receptors and are thus decisive whether rabbits serve as a model to study epitope masking in vivo. We observed a median concentration of 897±159 ng/ml enzymatically active DPP4 in 8 rabbits prior to immunization (Figure 6B), which was about 260 ng/ml higher compared to human samples. However, a substantially lower affinity for rbRBD (Kd 34.3) compared to hDPP4 (Kd 7.8 nM) was detected by Biolayer Interferometry (BLI) (Figure 6C). To evaluate how binding affinity and receptor concentration in the blood would impact epitope masking, the occupancy of the MERS-CoV spike assuming immunizations with 1 µg protein and a final tissue distribution volume of 3 ml was modelled. The model highlights that distinct affinities would have a major impact on epitope masking, as 47% versus 22% of MERS-CoV spike RBDs would be occupied by circulating human versus rabbit DPP4, respectively. In addition, masking of distinct amounts of immunogen in 2 volumes was modelled: 1 ml, which would be the actual i.m. vaccine application volume in rabbits, versus 30 ml (Figure 6E). According to the model, masking would be reduced if more than 4 µg of MERS-CoV spike would be applied in 1 ml application volume. This effect wanes the more the vaccine is distributed in the body, as no effect was observed of vaccine quantity on antigen occupancy modelling a final distribution volume of 30 ml. Collectively, the data suggest that rabbits represent a conservative in vivo model. If masking of a MERS-CoV vaccine would occur in rabbits, the effect would likely be multiplied in human.

### Example 6

To study epitope masking *in vivo,* rabbits were immunized intra-muscularly (i.m.) with 2 doses, 21 days apart, of 1 µg recombinant full-spike MERS-CoV wt versus the V555Y mutant (Experiment 1), OR a first dose of 5 µg recombinant full-spike MERS-CoV wt versus the V555Y mutant, followed by a second dose of 5 µg recombinant RBD MERS-CoV wt versus the V555Y mutant at day 21 (Experiment 2) (Figure 7A). In Experiment 1, the animals were immunized in 2 groups with 2 WT and 2 V555Y vaccinations in each group. All animals in the second group showed markedly decreased antibody titers compared to the first group, resulting in a higher variance of the WT versus V555Y conditions. Levels of circulating DPP4 were slightly increased after immunization, which may be attributed to the adjuvant as this effect was independent of DPP4 binding (Figure 7B). Spike (S1S2) and RBD specific antibody titers were highly increased for the V555Y mutant compared to wildtype, as indicated by a 5-fold higher median titer of RBD-specific IgG at 14 and 21 days post immunization (Figure 7C). Likewise, spike reactive IgG antibodies were increased by a factor of 19 (day 14) and 28 (day 21), and remained 6-fold increased 28 days post immunization. A similar tendency was observed in a pseudovirus neutralization assay (Figure 7D). In Experiment 2, spike (S1S2) specific IgG titers were 2.7-fold increased for V555Y compared to wt at day 28. Similarly, RBD specific IgG titers were increased 2.8-fold for V555Y compared to wt at day 28 (Figure 7E), which also resulted in higher pseudovirus neutralization titers (Figure 7F).

In summary, the data suggests that antibody responses elicited by a MERS-CoV RBD or spike-based vaccine may be improved by introduction of mutations that abrogates receptor binding.

**Example 7**

## Claims

1. Mutant receptor-binding domain (MERS-mRBD) of MERS-CoV (middle east respiratory syndrome coronavirus) or a fragment thereof and/or a mutant spike protein (MERS-mSpike) of MERS-CoV or a fragment thereof having a reduced binding strength to the RBD-receptor DPP4 (dipeptidylpeptidase 4) of MERS-CoV compared to a wild type receptor-binding domain of MERS-CoV (MERS-wtRBD) and/or having a reduced binding strength to sialic acid compared to a wild type spike of MERS-CoV (MERS-wtSpike).

2. MERS-mRBD or MERS-mSpike or the fragments thereof according to claim 1, wherein the MERS-mRBD, the MERS-mSpike or the fragments thereof exhibits a binding to anti-MERS-wtRBD neutralizing antibodies (anti-MERS-wtRBD-nABs).

3. MERS-mRBD or MERS-mSpike or the fragments thereof according to any of claims 1 to 2, wherein
(A) the MERS-mRBD or the fragment thereof comprises an amino acid sequence or a fragment thereof comprising one or more substitutions of amino acid residues at positions selected from S94, G96, S132, K136, R138, S139, L140, T146, E147, V168, G172, Y174, R176, W187, V189, A190, S191, or S193 of the MERS-wtRBD of SEQ ID NO: 1 (corresponding to S460, G462, S498, K502, S504, R505, L506, T512, E513, V534, G538, Y540, R542, W553, V555, A556, S557, or S559 of the MERS-wtSpike of SEQ ID NO: 46) or the fragment thereof, wherein the MERS-mRBD or the fragment thereof has, except for the substitutions, an amino acid sequence identity of 85% or more to SEQ ID NO: 1; and/or
(B) the MERS-mSpike or the fragment thereof comprises more preferably consists of, an amino acid sequence or a fragment thereof comprising one or more substitutions of amino acid residues at positions selected from F39, S133, R307, H91 and/or one or more substitutions of amino acid residues enabling an oligomerization of two or more mSpikes, and/or enabling one or more disulphide bonds, preferably a combination of three substitutions of amino acid residues at positions V458, V1060 and L1061 (VPP) and/or a combination of two substitutions at positions T424 and S459 (TS) of the MERS-wtSpike of SEQ ID NO: 46 or the fragment thereof, wherein the MERS-mSpike or the fragment thereof has, except for the substitutions, an amino acid sequence identity of 80% or more to SEQ ID NO: 46.

4. MERS-mRBD or MERS-mSpike or the fragments thereof according to any one of the preceding claims,
(A) wherein, for the MERS-mRBD or the fragment thereof, the substitution of the amino acid residue at the position:
- S94 is S94W;
- G96 is selected from G96W, G96Y, G96R, G96F, G96L, G96Q, preferably from G96R, G96L, G96Q ;
- S132 is S132W;
- K136 is selected from K136D, K136E, K136G, K136Y, K136S, K136P;
- S138 is S138W;
- R139 is R139L;
- L140 is selected from L140Y, L140W, L140R, L140H, preferably L140 is L140R;
- T146 is T146A;
- E147 is selected from E147Y, E147W, E147R, E147F;
- V168 is V168W;
- G172 is selected from G172C, G172D, G172E, G172F, G172H, G172I, G172K, G172L, G172N, G172Q, G172R, G172T, G172V, G172W, G172A, G172M, G172P, G172S, G172Y, preferably from G 172H, G172W, G172E, G172K, G172L, G172Y, G172Q, G172T, G172R or G172D, more preferably from G172E, G172K; G172L, G172Y, G172Q, G172T, G172R, or G172D, more preferably from G172E or G172K; most preferably from G172K;
- Y174 is Y174C;
- R176 is R176G;
- W187 is selected from W187A, W187C, W187D, W187E, W187G, W187K, W187L, W187M, W187N, W187P, W187Q, W187R, W187S, W187T, W187V
- V189 is selected from V189W, V189Y, V189F, V189R, V189K, V189A, preferably from V189W, V189Y, V189F, V189R, more preferably V189 is V189Y;
- A190 is A190W;
- S191 is selected from S191Y, S191W, S191F;
- S193 is S193W and/or
(B) wherein, for the MERS-mSpike or the fragment thereof, the substitution of the amino acid residue at the position:
- F39 is F39A
- S133 is S133A
- R307 is R307A
- H91 is H91A
- V458 is V458C
- V1060 is V1060K
- L1061 is L1061C
- T424 is T424C
- S459 is S459C.

5. MERS-mRBD or MERS-mSpike or the fragments thereof according to any one of the preceding claims,
(A) wherein the MERS-mRBD or the fragment thereof comprises an amino acid sequence or a fragment thereof comprising one or more substitutions of amino acid residues at positions selected from G96, L140, G172, or V189, of the MERS-wtRBD of SEQ ID NO: 1 (corresponding to G462, L506, G538, or V555 of the MERS-wtSpike of SEQ ID NO:45), or the fragment thereof, preferably L140, G172, or V189 or the fragment thereof, more preferably the substitution of the amino acid residue at the position
- G96 is selected from G96R, G96Q, G96L,
- L140 is selected from L140R,
- G172 is selected from G 172H, G172W, G172E, G172K, G172L, G172Y, G172Q, G172T, G172R or G172D,
- V189 is selected from V189W, V189Y, V189R, V189F; and/or
(B) the MERS-mSpike or the fragment thereof comprises an amino acid sequence or a fragment thereof comprising a combination of substitutions of amino acid residues at positions V458, V1060 and L1061 (VPP) or a combination of substitutions of amino acid residues at positions T424 and S459 (TS) of the MERS-wtSpike of SEQ ID NO: 46 or the fragment thereof, preferably, the substitution of the amino acid residue at the position
- V458 is V458C
- V1060 is V1060K
- L1061 is L1061C
- T424 is T424C
- S459 is S459C.

6. MERS-mRBD or MERS-mSpike or the fragments thereof according to any one of the preceding claims,
(A) wherein the MERS-mRBD or the fragment thereof comprises an amino acid sequence or a fragment thereof comprising one or more substitutions of amino acid residues at positions selected from, L140, G172, or V189, of the MERS-wtRBD of SEQ ID NO: 1 (corresponding to, L506, G538, or V555, of the MERS-wtSpike of SEQ ID NO: 46) or the fragment thereof, wherein preferably, the substitution of the amino acid residue at the position
- L 140 is L140R;
- G172 is G172E, G172K; G172L, G172Y, G172Q, G172T, G172R, or G172D;
- V189 is V189W, V189Y, V189R, V189F; and/or
(B) the MERS-mSpike or the fragment thereof comprises an amino acid sequence or a fragment thereof comprising a combination of substitutions of amino acid residues at positions T424 and S459 (TS) of the MERS-wtSpike of SEQ ID NO: 46 or the fragment thereof, preferably, the substitution of the amino acid residue at the position
- T424 is T424C
- S459 is S459C.

7. MERS-mRBD or fragment thereof according to any one of the preceding claims, wherein the MERS-mRBD or the fragment thereof comprises an amino acid sequence or a fragment thereof comprising one or more substitutions of amino acid residues at positions selected from, L140, G172, or V189, of the MERS-wtRBD of SEQ ID NO: 1 (corresponding to, L506, G538, or V555, of the MERS-wtSpike of SEQ ID NO: 45) or the fragment thereof, preferably selected from L140R, G172E, G172K, V189W, V189Y, V189R, V189F, more preferably selected from L140R, G172K, V189Y.

8. MERS-mRBD or MERS-mSpike or fragments thereof according to any one of the preceding claims, wherein
(A) the MERS-mRBD or the fragment thereof comprises an amino acid sequence having a sequence identity of 85% or more to an amino acid sequence selected from SEQ ID NO: 2 , SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37 ; SEQ ID NO: 38, SEQ ID NO: 39, SEQ ID NO: 40, SEQ ID NO: 41, SEQ ID NO: 42, SEQ ID NO: 43, SEQ ID NO: 44; SEQ ID NO: 45; and/or
(B) the MERS-mSpike or the fragment thereof comprises an amino acid sequence having a sequence identity of 80% or more to an amino acid sequence selected from SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56.

9. MERS-mRBD or MERS-mSpike or fragments thereof to any one of the preceding claims, wherein
(A) the MERS-mRBD or the fragment thereof comprises an amino acid sequence having a sequence identity of 85% or more to an amino acid sequence selected from SEQ ID NO: 3, SEQ ID NO: 4, SEQ ID NO: 5, SEQ ID NO: 6, SEQ ID NO: 7, SEQ ID NO: 8, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37; SEQ ID NO: 38 and/or
(B) the MERS-mSpike or the fragment thereof comprises an amino acid sequence having a sequence identity of 80% or more to an amino acid sequence selected from SEQ ID NO: 47, or SEQ ID NO: 48.

10. MERS-mRBD or MERS-mSpike or fragments thereof to any one of the preceding claims, wherein the MERS-mRBD or the fragment thereof comprises an amino acid sequence having a sequence identity of 85% or more to an amino acid sequence selected from SEQ ID NO: 11, SEQ ID NO: 17, SEQ ID NO: 18, SEQ ID NO: 34, SEQ ID NO: 35, SEQ ID NO: 36, SEQ ID NO: 37, or SEQ ID NO: 38.

11. MERS-mSpike or a fragment thereof according to anyone of the preceding claims comprising the MERS-mRBD or the fragment thereof according to any of claims 1 to 10.

12. Polypeptide or protein comprising the MERS-mRBD or the fragment thereof or the MERS-mSpike or the fragment thereof according to any of claims 1 to 11.

13. Nucleic acid comprising a nucleotide sequence encoding for:
- the MERS-mRBD or the fragment thereof according to any of claims 1 to 10;
- the MERS-mSpike or the fragment thereof according to any of claims 1 to 11; or
- the polypeptide or protein according to claim 12.

14. Vaccine composition comprising as an active ingredient:
- one or more MERS-mRBDs or the fragments thereof according to any of claims 1 to 10; and/or
- one or more MERS-mSpikes or the fragments thereof according to claims 1 to 11; and/or
- one or more polypeptides or proteins according to claim 12; and/or
- one or more nucleic acids according to claim 13.

15. One or more MERS-mRBDs or the fragments thereof according to any of claims 1 to 10; and/or
one or more MERS-mSpikes or the fragments according to claims 1 to 11; and/or
one or more polypeptides or proteins according to claim 12; and/or
one or more nucleic acids according to any of claims 13; and/or
the vaccine composition according to claim 14,
for use in the prevention and/or treatment of diseases caused by MERS-CoV in a subject.
